# EUROPEAN PATENT APPLICATION

(11) **EP 4 056 704 A1**
(43) Date of publication of application: **14.09.2022**
(21) Application number: 20889958.3
(22) Date of filing: 16.11.2020
(51) Int. Cl.: C12N 15/12, G01N 33/15, G01N 33/50

(54) **CAPTURE METHOD FOR CANDIDATE SUBSTANCES THAT CAN BIND WITH CONJUGATE OF MR1 PROTEIN AND ?2-MICROGLOBULIN PROTEIN, METHOD FOR MANUFACTURING CANDIDATE SUBSTANCE THAT CAN BIND WITH CONJUGATE OF MR1 PROTEIN AND ?2-MICROGLOBULIN PROTEIN, AND METHOD FOR MANUFACTURING MAIT CELL LIGAND CANDIDATE SUBSTANCE**

(30) Priority: 22.11.2019 JP 2019211860
(71) Applicant: OSAKA UNIVERSITY, Suita-shi Osaka 565-0871 (JP)
(72) Inventor: MOTOZONO Chihiro, Suita-shi, Osaka 565-0871 (JP); YAMASAKI Sho, Suita-shi, Osaka 565-0871 (JP)
(74) Representative: J A Kemp LLP
(86) International application number: PCT/JP2020/042605
(87) International publication number: WO 2021/100653

(57) **Abstract**

The present invention provides a method for capturing a candidate substance that can bind to a complex of a MR1 protein and a β2 microglobulin protein more widely. A method for capturing a candidate substance that can bind to a complex of a MR1 protein and a β2 microglobulin protein of the present invention includes steps of: forming a complex of the test substance, the MR1 protein, and the β2 microglobulin protein by causing the test substance, the MR1 protein, and the β2 microglobulin protein to coexist; and reducing the complex with a reducing agent.

## Description

### TECHNICAL FIELD

The present invention relates to a method for capturing a candidate substance that can bind to a complex of a MR1 protein and a β2 microglobulin protein, a method for producing a candidate substance that can bind to a complex of a MR1 protein and a β2 microglobulin protein, and a method for producing a ligand candidate substance for a MAIT cell.

### BACKGROUND ART

Mucosal-associated invariant T (MAIT) cells were identified initially as T cells present in the intestinal tract. Then, the MAIT cells have been found to be present throughout a body, including mucosal tissues, suggesting that they contribute to infectious and autoimmune diseases. T-cell receptors (TCRs) in MAIT cells are thought to recognize and activate metabolites derived from the intestinal flora and the like, presented on a complex of a MHC class I-related protein 1 (MR1 protein) and a β2 microglobulin (β2m) protein (Non-Patent Literature 1).

### Citation List

### Non-Patent Literature

Non-Patent Literature 1: Dale I. Godfrey et.al., "The biology and functional importance of MAIT cells", Nature Immunogolg, 2019, vol.20, pages 1110-1128

### SUMMARY OF INVENTION

### Technical Problem

Since the MR1 protein and the β2m protein form the complex in a ligand dependent manner, the presence or absence of the ligand causes a difference between forming or not forming the complex. Therefore, ligands for MAIT cells are screened using this phenomenon. Vitamins and their metabolites (hereinafter also collectively referred to as "vitamin metabolites") are identified as ligands for MAIT cells. However, in the method using the formation of the complex as an indicator, a ligand other than a vitamin metabolite, such as an endogenous ligand or the like, or a candidate substance of such a ligand has not yet been identified.

With the foregoing in mind, it is an object of the present invention to provide a method for capturing a candidate substance that can bind to the complex more widely.

### Solution to Problem

In order to achieve the above object, the present invention provides a method for capturing a candidate substance that can bind to a complex of a MR1 protein and a β2 microglobulin protein (hereinafter, also referred to as a "capturing method"), including steps of: forming a complex of the test substance, the MR1 protein, and the β2 microglobulin protein by causing a test substance, a MR1 protein, and a β2 microglobulin protein to coexist; and reducing the complex with a reducing agent.

The present invention also provides a method for producing a candidate substance that can bind to a complex of a MR1 protein and a β2 microglobulin protein (hereinafter, also referred to as a "candidate substance producing method"), including steps of: forming a reduced complex of a reduced test substance, MR1 protein, and β2 microglobulin protein obtained by reducing a test substance, a MR1 protein, and a β2 microglobulin protein; and detecting a test substance in the reduced complex, wherein the forming a reduced complex is carried out by the method for capturing a candidate substance according to the present invention.

The present invention also provides a method for producing a ligand candidate substance for a MAIT cell (hereinafter, also referred to as a "ligand candidate substance producing method"), including steps of: detecting a candidate substance that binds to a complex of a MR1 protein and a β2 microglobulin protein from a test substance; contacting the candidate substance detected in the detecting, the MR1 protein, and the β2 microglobulin protein with a MAIT cell; and selecting a ligand candidate substance for the MAIT cell based on activation of the MAIT cell in the contacting, wherein the detecting is carried out by the candidate substance producing method according to the present invention.

The present invention also provides a kit for capturing a candidate substance that can bind to a complex of a MR1 protein and a β2 microglobulin protein (hereinafter, also referred to as a "capturing kit"), including: a MR1 protein; a β2 microglobulin protein; and a reducing agent, wherein the kit is used in the capturing method according to the present invention. Advantageous Effects of Invention

According to the present invention, a candidate substance that can bind to the complex can be captured more widely. For this reason, according to the present invention, for example, a ligand other than a vitamin metabolite or a candidate substance of such a ligand can be identified.

### BRIEF DESCRIPTION OF DRAWINGS

[FIGs. 1A and 1B] FIGs. 1A and 1B are graphs showing the thermodynamic stability of the complex in Example 1.
[FIG. 2] FIG. 2 is a graph showing the data having different (or no) peaksdepending on with or without the reduction treatment in Example 2.
[FIG. 3] FIG. 3 is a graph showing the data having different peak area sizes depending on with or without the reduction treatment in Example 2.

### DESCRIPTION OF EMBODIMENTS

### <Capturing method>

As described above, the method for capturing a candidate substance that can bind to a complex of a MR1 protein and a β2 microglobulin protein of the present invention includes the steps of: forming a complex of the test substance, the MR1 protein, and the β2 microglobulin protein by causing a test substance, a MR1 protein, and a β2 microglobulin protein to coexist; and reducing the complex with a reducing agent. The capturing method of the present invention is characterized in that, the reducing the complex is performed after the forming a complex, and other steps and conditions are not particularly limited. In the capturing method of the present invention, for example, by reducing the complex in the reducing, a test substance contributing to the formation of the complex can be immobilized in a state of being captured in the complex. Thus, according to the capturing method of the present invention, a candidate substance that can bind to the complex can be captured more widely.

The inventors of the present invention considered that, a part of the substance (candidate substance) contributing to the formation of the complex may have a relatively weak binding force to the complex as compared to a ligand for a MAIT cell (vitamin B9 (folic acid, vitamin M), diclofenac (2-(2-(6-dichlorophenylamino)phenyl)acetic acid), N-(6-formyl-3, 4-dihydro-4-oxo-2-pteridinyl)-acetamide (Ac6FP), 5-(2-oxopropylideneamino)-6-D-ribitylaminouracil (5-OP-RU), etc.)) in the method for identifying a ligand for a MAIT cell using the formation of the complex, and therefore the candidate substance may be detached from the complex in the process of the identification method, resulting in no ligand other than a vitamin metabolite being able to be identified. As a result of an intensive study, the inventors of the present invention have found that a candidate substance that can bind to the complex can be captured more widely by subjecting a formed complex to a reduction treatment, and have completed the present invention. It is presumed that, in the capturing method of the present invention, by subjecting the complex to a reduction treatment after being formed with the test substance, the candidate substance can be immobilized in a state of being held in the complex, so that the candidate substance can be prevented from falling off, and thereby, the candidate substance that can bind to the complex can be captured more widely. However, the present invention is not limited in any way to this presumption. Since a candidate substance that can bind to the complex can be recovered, collected, or extracted from a test substance, the capturing method of the present invention can also be referred to as, for example, a recovery method, a collection method, or an extraction method. The candidate substance that can bind to the complex can also be referred to as, for example, a candidate substance that can form the complex. In addition, since the capturing method of the present invention can more stably capture a candidate substance that can bind to the complex, it can also be referred to as a method for stabilizing a complex, a method for stabilizing a candidate substance captured in a complex, a method for stably capturing a candidate substance in a complex, a method for stabilizing a candidate substance induced to form a complex, and the like.

In the present invention, the "capture" means, for example, a state of catching or capturing a target. Specifically, the "capture" means, for example, holding the candidate substance being integrated with the complex. Therefore, the "capture" may also be referred to as "hold", "maintain", "grasp", or the like, for example.

The MR1 protein is a non-classical MHC-class I molecule. The origin of the MR1 protein is not particularly limited, and examples thereof include humans and non-human animals excluding humans. Examples of the non-human animal include a mouse, a rat, a dog, a monkey, a rabbit, a sheep, a horse, a guinea pig, and a cat.

Examples of the MR1 protein include the following proteins. Human-derived MR1 protein may be, for example, isoforms A to D, and is preferably isoform A. The human MR1 protein isoform A (hMR1A) may be, for example, a protein formed from an amino acid sequence (SEQ ID NO: 1) registered under GenBank Accession No. AAC50174. A polynucleotide encoding hMR1A may be, for example, a polynucleotide formed from a base sequence registered under GenBank Accession No. U22963. The human MR1 protein isoform B (hMRIB) may be, for example, a protein formed from an amino acid sequence (SEQ ID NO: 2) registered under GenBank Accession No. AAD01442. A polynucleotide encoding hMRIB may be, for example, a polynucleotide formed from a base sequence registered under GenBank Accession No. AF010446. The human MR1 protein isoform C (hMRIC) may be, for example, a protein formed from an amino acid sequence (SEQ ID NO: 3) registered under GenBank Accession No. AAD01443. A polynucleotide encoding hMRIC may be, for example, a polynucleotide formed from a base sequence registered under GenBank Accession No. AF010447. The human MR1 protein isoform D (hMR1D) may be, for example, a protein formed from an amino acid sequence (SEQ ID NO: 4) registered under GenBank Accession No. AAD01933. A polynucleotide encoding hMR1D may be, for example, a polynucleotide formed from a base sequence registered under GenBank Accession No. AF031469. In the amino acid sequence of SEQ ID NO: 1, the amino acid sequences shown in parentheses indicate a signal peptide, an extracellular domain, and an intracellular domain in the order from the N-terminal side. In the amino acid sequences of SEQ ID NOs: 2 to 4, the amino acid sequences shown in parentheses indicate the signal peptide and the extracellular domain in the order from the N-terminal side. Note that, in the MR1 protein, the extracellular domain forms a complex with, for example, the β2m protein.
Human MR1 protein isoform A (hMR1A, SEQ ID NO: 1) 
[MGELMAFLLPLIIVLMVKHSDS][RTHSLRYFRLGVSDPIHGVPEFISVGYVDSHPITTYDS
VTRQKEPRAPWMAENLAPDHWERYTQLLRGWQQMFKVELKRLQRHYNHSGSHTYQR
MIGCELLEDGSTTGFLQYAYDGQDFLIFNKDTLSWLAVDNVAHTIKQAWEANQHELLYQ
KNWLEEECIAWLKRFLEYGKDTLQRTEPPLVRVNRKETFPGVTALFCKAHGFYPPEIYMT
WMKNGEEIVQEIDYGDILPSGDGTYQAWASIELDPQSSNLYSCHVEHCGVHMVLQVPQ][
ESETIPLVMKAVSGSIVLVIVLAGVGVLVWRRRPREQNGAIYLPTPDR]
Human MR1 protein isoform B (hMRIB, SEQ ID NO: 2) 
[MGELMAFLLPLIIVLMVKHSDS][RTHSLRYFRLGVSDPIHGVPEFISVGYVDSHPITTYDS
VTRQKEPRAPWMAENLAPDHWERYTQLLRGWQQMFKVELKRLQRHYNHSGSHTYQR
MIGCELLEDGSTTGFLQYAYDGQDFLIFNKDTLSWLAVDNVAHTIKQAWEANQHELLYQ
KNWLEEECIAWLKRFLEYGKDTLQRTESETIPLVMKAVSGSIVLVIVLAGVGVLVWRRRP
REQNGAIYLPTPDR]
Human MR1 protein isoform C (hMRIC, SEQ ID NO: 3) 
[MVKHSDS][RTHSLRYFRLGVSDPIHGVPEFISVGYVDSHPITTYDSVTRQKEPRAPWMA
ENLAPDHWERYTQLLRGWQQMFKVELKRLQRHYNHSGSHTYQRMIGCELLEDGSTTG
FLQYAYDGQDFLIFNKDTLSWLAVDNVAHTIKQAWEANQHELLYQKNWLEEECIAWLK
RFLEYGKDTLQRTGKEKEKASFPHCLNNCFYT]
Human MR1 protein isoform D (hMR1D, SEQ ID NO: 4)

[MAFLLPLIIVLMVKHSDS][RTHSLRYFRLGVSDPIHGVPEFISVGYVDSHPITTYDSVTRQ
KEPRAPWMAENLAPDHWERYTQLLRGWQQMFKVELKRLQRHYNHSGSHTYQRMIGC
ELLEDGSTTGFLQYAYDGQDFLIFNKDTLSWLAVDNVAHTIKQAWEANQHELLYQKNW
LEEECIAWLKRFLEYGKDTLQRTESETIPLVMKAVSGSIVLVIVLAGVGVLVWRRRPREQ
NGAIYLPTPDR]

The mouse-derived MR1 protein may be, for example, isoform A. The murine MR1 protein isoform A (mMR1A) may be, for example, a protein formed from an amino acid sequence (SEQ ID NO: 5) registered under GenBank Accession No. AAD01444. A polynucleotide encoding mMR1A may be, for example, a polynucleotide formed from a base sequence registered under GenBank Accession No. AF010448. In the amino acid sequence of SEQ ID NO: 5, the amino acid sequences shown in parentheses indicate a signal peptide, an extracellular domain, and an intracellular domain in the order from the N-terminal side.
Murine MR1 protein isoform A (mMRIA, SEQ ID NO: 5)

[MMLLLPLLAVFLVKRSHT][RTHSLRYFRLAVSDPGPVVPEFISVGYVDSHPITTYDSVTR
QKEPKAPWMAENLAPDHWERYTQLLRGWQQTFKAELRHLQRHYNHSGLHTYQRMIG
CELLEDGSTTGFLQYAYDGQDFIIFNKDTLSWLAMDYVAHITKQAWEANLHELQYQKN
WLEEECIAWLKRFLEYGRDTLERTEHPVVRTTRKETFPGITTFFCRAHGFYPPEISMTWM
KNGEEIAQEVDYGGVLPSGDGTYQTWLSVNLDPQSNDVYSCHVEHCGRQMVLEAP][R
ESGDILRVSTISGTTILIIALAGVGVLIWRRSQELKEVMYQPTQVNEGSSPS]

The MR1 protein may be a soluble MR1 protein. The soluble MR1 protein may be, for example, a MR1 protein comprising an extracellular domain in the human-derived MR1 protein and the mouse-derived MR1 protein. Specific examples of the soluble MR1 protein include the soluble hMR1A to D and the soluble mMR1A described below.
Soluble hMR1A (SEQ ID NO: 6) 
RTHSLRYFRLGVSDPIHGVPEFISVGYVDSHPITTYDSVTRQKEPRAPWMAENLAPDHW
ERYTQLLRGWQQMFKVELKRLQRHYNHSGSHTYQRMIGCELLEDGSTTGFLQYAYDGQ
DFLIFNKDTLSWLAVDNVAHTIKQAWEANQHELLYQKNWLEEECIAWLKRFLEYGKDT
LQRTEPPLVRVNRKETFPGVTALFCKAHGFYPPEIYMTWMKNGEEIVQEIDYGDILPSGD
GTYQAWASIELDPQSSNLYSCHVEHCGVHMVLQVPQ
Soluble hMRIB (SEQ ID NO: 7) 
RTHSLRYFRLGVSDPIHGVPEFISVGYVDSHPITTYDSVTRQKEPRAPWMAENLAPDHW
ERYTQLLRGWQQMFKVELKRLQRHYNHSGSHTYQRMIGCELLEDGSTTGFLQYAYDGQ
DFLIFNKDTLSWLAVDNVAHTIKQAWEANQHELLYQKNWLEEECIAWLKRFLEYGKDT
LQRTESETIPLVMKAVSGSIVLVIVLAGVGVLVWRRRPREQNGAIYLPTPDR
Soluble hMRIC (SEQ ID NO: 8) 
RTHSLRYFRLGVSDPIHGVPEFISVGYVDSHPITTYDSVTRQKEPRAPWMAENLAPDHW
ERYTQLLRGWQQMFKVELKRLQRHYNHSGSHTYQRMIGCELLEDGSTTGFLQYAYDGQ
DFLIFNKDTLSWLAVDNVAHTIKQAWEANQHELLYQKNWLEEECIAWLKRFLEYGKDT
LQRTGKEKEKASFPHCLNNCFYT
Soluble hMR1D (SEQ ID NO: 9) RTHSLRYFRLGVSDPIHGVPEFISVGYVDSHPITTYDSVTRQKEPRAPWMAENLAPDHW 
ERYTQLLRGWQQMFKVELKRLQRHYNHSGSHTYQRMIGCELLEDGSTTGFLQYAYDGQ
DFLIFNKDTLSWLAVDNVAHTIKQAWEANQHELLYQKNWLEEECIAWLKRFLEYGKDT
LQRTESETIPLVMKAVSGSIVLVIVLAGVGVLVWRRRPREQNGAIYLPTPDR
Soluble mMRIA (SEQ ID NO: 10)

RTHSLRYFRLAVSDPGPVVPEFISVGYVDSHPITTYDSVTRQKEPKAPWMAENLAPDHW
ERYTQLLRGWQQTFKAELRHLQRHYNHSGLHTYQRMIGCELLEDGSTTGFLQYAYDGQ
DFIIFNKDTLSWLAMDYVAHITKQAWEANLHELQYQKNWLEEECIAWLKRFLEYGRDT
LERTEHPVVRTTRKETFPGITTFFCRAHGFYPPEISMTWMKNGEEIAQEVDYGGVLPSGD
GTYQTWLSVNLDPQSNDVYSCHVEHCGRQMVLEAP

The MR1 protein may be a mutated MR1 protein in which one or several amino acids are deleted, substituted, inserted and/or added, in the amino acid sequence of each MR1 protein. The mutated MR1 protein may be, for example, a protein formed from an amino acid sequence in which an underlined amino acid (amino acid to be mutated) is substituted for another amino acid in the human-derived MR1 protein and the mouse-derived MR1 protein.

In the amino acid sequences of the hMR1A to D and mMR1A, the amino acids to be mutated may be, lysines (K) at positions 65, 65, 50, 61, and 61 (lysine to be mutated), which are underlined, respectively. By introducing a mutation into the lysine to be mutated, the mutated hMR1A to D and mMRIA can form complexes with β2m proteins, for example, in a ligand-independent manner. Thus, a MR1 protein in which a mutation is introduced into a lysine to be mutated can also capture a candidate substance having a lower binding property to a complex of a MR1 protein and a β2m protein, for example. Lysines to be mutated in other animal-derived MR1 proteins can be identified, for example, by alignment with hMR1A to D and/or mMRIA.

In the amino acid sequence of hMR1A, the amino acid to be mutated is, for example, cysteine (C) at position 283 (cysteine to be mutated), which is underlined. By introducing a mutation into the cysteine (C) at position 283, the mutated hMR1A can improve, for example, stability of the mutated hMR1A. Therefore, the mutated hMR1A can produce a recombinant protein more stably. In the amino acid sequence of mMR1A, the amino acid to be mutated is, for example, cysteine (C) at position 279, which is underlined. By introducing a mutation into the cysteine (C) at position 279, the mutated mMRIA can improve, for example, stability of the mutated hMR1A. Therefore, the mutated mMRIA can produce a recombinant protein more stably. Cysteines to be mutated in other animal-derived MR1 proteins can be identified, for example, by alignment with hMR1A to D and/or mMRIA.

The other amino acid may be, for example, an amino acid residue having a side chain of properties not similar to those of the amino acid to be substituted, and is specifically, for example, alanine.

The MR1 protein may be a soluble mutated MR1 protein. In this case, in the soluble hMR1A to D and soluble mMR1A, the soluble mutated MR1 protein can be prepared, for example, by introducing mutations into amino acids at positions corresponding to the mutated amino acids in the hMR1A to D and mMRIA.

The amino acid sequences of the hMR1A to D and mMRIA may or may not have a signal peptide. When using an expression cell of a MR1 protein as a MR1 protein, the MR1 protein preferably has the signal peptide. On the other hand, when a crude extract, a crude purified product, a purified product, or a recombinant protein containing a MR1 protein is used as the MR1 protein, the MR1 protein preferably does not have the signal peptide.

The MR1 protein may be, for example, a protein (polypeptide) formed from an amino acid sequence of (M1), (M2) or (M3) below:
(M1) a protein formed from an amino acid sequence of a MR1 protein derived from various animals;
(M2) a protein formed from an amino acid sequence in which one or several amino acids are deleted, substituted, inserted and/or added, in the amino acid sequence of the (M1), and that can form a complex with a β2 microglobulin protein; or
(M3) a protein formed from an amino acid sequence having a 50% or more identity to the amino acid sequence of the (M1), and that can form a complex with a β2 microglobulin protein.

In the (M1), the amino acid sequence of a MR1 protein derived from various animals may be, for example, an amino acid sequence of any of SEQ ID NOs: 1 to 5. The MR1 proteins derived from animals other than the human and mouse may be identified using homology of amino acid sequences, for example, based on the amino acid sequence of at least one of the human MR1 protein and the murine MR1 protein. The analysis of the MR1 proteins derived from animals other than the human and mouse can be carried out, for example, by using an amino acid sequence of at least one of the human MR1 protein or the murine MR1 protein, as a reference sequence, and analysis software such as BLAST, FASTA, and the like.

In the (M2), the "one or several" may be, for example, a range in which the (M2) can form a complex with the β2 microglobulin protein, that is, a range in which the complex can be formed when causing the (M2) to coexist with the β2 microglobulin protein and a ligand for a MAIT cell (e.g., 5-(2-oxopropylideneamino)-6-D-ribitylaminouracil (5-OP-RU)). The "one or several" in the (M2) are, for example, 1 to 150, 1 to 135, 1 to 120, 1 to 105, 1 to 90, 1 to 75, 1 to 60, 1 to 45, 1 to 30, 1 to 20, 1 to 10, 1 to 9, 1 to 5, 1 to 3, or 1 or 2, in the amino acid sequence of the (M1).

In the (M3), for example, the "identity" may be determined in a range in which the (M3) can form a complex with a β2 microglobulin protein, that is, a range in which the complex can be formed when causing the (M3) to coexist with the β2 microglobulin protein and the ligand for a MAIT cell. The "identity" of the (M3) is, for example, 50% or more, 55% or more, 60% or more, 65% or more, 70% or more, 75% or more, 80% or more, 85% or more, 90% or more, 95% or more, 96% or more, 97% or more, 98% or more, or 99% or more, relative to the amino acid sequence of the (M1). The identity can be calculated, for example, by default parameters using analysis software such as BLAST, FASTA, and the like.

In the (M1), the MR1 protein derived from various animals may be, for example, a soluble MR1 protein derived from various animals, a mutated MR1 protein derived from various animals, or a soluble and mutated MR1 protein derived from various animals.

When the MR1 protein derived from various animals in the (M1) is a mutated MR1 protein derived from various animals and the mutated amino acid in the mutated MR1 protein is a lysine to be mutated, the "one or several" in the (M2) may be, for example, a range in which the (M2) can form a complex with a β2 microglobulin protein, that is, a range in which the complex can be formed when causing the (M2) to coexist with the β2 microglobulin protein. In addition, in this case, the "identity" in the (M3) may be, for example, a range in which the (M3) can form a complex with a β2 microglobulin protein, that is, a range in which the complex can be formed when causing the (M3) to coexist with the β2 microglobulin protein.

A conservative amino acid substitution may be introduced into the MR1 protein, for example. The conservative amino acid substitution means, for example, a substitution by an amino acid residue having a side chain of properties similar to those of an amino acid to be substituted.

The MR1 protein may be, for example, a polypeptide including a partial sequence of a MR1 protein. In this case, a polypeptide made of the partial sequence can form the complex when causing the polypeptide to coexist with a β2 microglobulin protein and ligand for a MAIT cell.

In the present invention, the MR1 protein comprises, for example, a natural amino acid, a non-natural amino acid and/or a modified natural amino acid, and is preferably comprises a natural amino acid and/or a modified natural amino acid. The amino acid constituting the MR1 protein may be, for example, an L body, a D body, or both, and is preferably an L body.

The MR1 protein may be an unmodified protein or a modified protein. Examples of the modification include: addition of functional groups, such as acylation, acetylation, alkylation, amidation, biotinylation, formylation, γ-carboxylation, glutamination, glycosylation, glycylation, isoprenylation, phosphorylation, PEGylation, racemization, and the like; and intraprotein crosslinking such as formation of disulfide bonds, and the like.

The β2m protein is one of the proteins constituting the MHC class I molecule. The origin of the β2m protein is not particularly limited, and examples thereof include humans and non-human animals excluding humans. Examples of the non-human animal include a mouse, a rat, a dog, a monkey, a rabbit, a sheep, a horse, a guinea pig, and a cat. The β2m protein may be, for example, homologous or heterologous to the MR1 protein.

As the β2m protein, the following proteins can be exemplified. The human-derived β2m protein (hβ2m) may be, for example, a protein formed from an amino acid sequence (SEQ ID NO: 11) registered under GenBank Accession No. BAA35182. A polynucleotide encoding hβ2m may be, for example, a polynucleotide formed from a base sequence registered under GenBank Accession No. AB021288. In the amino acid sequence of SEQ ID NO: 11, the amino acid sequences shown in parentheses indicate the signal peptide and the extracellular domain in the order from the N-terminal side.
Human β2m protein (hβ2m, SEQ ID NO: 11)

[

M]

The mouse-derived β2 microglobulin protein (mβ2m) may be, for example a protein formed from an amino acid sequence (SEQ ID NO: 12) registered under GenBank Accession No. NP_033865. A polynucleotide encoding mβ2m may be, for example, a polynucleotide formed from a base sequence registered under GenBank Accession No. NM_009735. In the amino acid sequence of SEQ ID NO: 12, the amino acid sequences shown in parentheses indicate the signal peptide and the extracellular domain in the order from the N-terminal side.
Mouse β2m protein (mβ2m, SEQ ID NO: 12)

[MARSVTLVFLVLVSLTGLYA][IQKTPQIQVYSRHPPENGKPNILNCYVTQFHPPHIEIQML
KNGKKIPKVEMSDMSFSKDWSFYILAHTEFTPTETDTYACRVKHASMAEPKTVYWDRD
M]

The β2m protein may be a soluble β2m protein, for example. The soluble β2m protein may be, for example, in the human-derived β2m protein and the mouse-derived β2m protein, a β2m protein from which a signal peptide is deleted, i.e., a β2m protein formed from an extracellular domain. Specific examples of the soluble β2m protein include the soluble hβ2m and the soluble mβ2m below.
Soluble hβ2m (SEQ ID NO: 13)
Soluble mβ2m (SEQ ID NO: 14)

The β2m protein may be, for example, a protein (polypeptide) formed from an amino acid sequence of (B1), (B2) or (B3) below.
(B1) a protein formed from an amino acid sequence of a β2m protein derived from various animals;
(B2) a protein formed from an amino acid sequence in which one or several amino acids are deleted, substituted, inserted and/or added, in the amino acid sequence of the (B1), and that can form a complex with a MR1 protein; or
(B3) a protein formed from an amino acid sequence having 50% or more identity to the amino acid sequence of the (B1) and that can form a complex with a MR1 protein.

In the (B1), the amino acid sequence of a β2m protein derived from various animals may be, for example, any amino acid sequence of SEQ ID NOs: 11 to 12. The β2m proteins derived from animals other than the human and mouse may be identified using homology of amino acid sequences, for example, based on the amino acid sequence of at least one of the human β2m protein or the murine β2m protein. The analysis of the β2m proteins derived from animals other than the human and mouse can be carried out, for example, using an amino acid sequence of at least one of the human β2m protein or the murine β2m protein, as a reference sequence, and analysis software such as BLAST, FASTA, and the like.

In the (B2), the "one or several" may be, for example, a range in which the (B2) can form a complex with the MR1 protein, that is, a range in which the complex can be formed when causing the (B2) to coexist with the MR1 protein and ligand for the MAIT cell. The "one or several" in the (B2) are, for example, 1 to 150, 1 to 135, 1 to 120, 1 to 105, 1 to 90, 1 to 75, 1 to 60, 1 to 45, 1 to 30, 1 to 20, 1 to 10, 1 to 9, 1 to 5, 1 to 3, or 1 or 2, in the amino acid sequence of the (B1).

In the (B3), for example, the "identity" may be determined in a range in which the (B3) can form a complex with a MR1 protein, that is, a range in which the complex can be formed when causing the (B3) to coexist with the MR1 protein and the ligand for a MAIT cell. The "identity" of the (B3) is, for example, 50% or more, 55% or more, 60% or more, 65% or more, 70% or more, 75% or more, 80% or more, 85% or more, 90% or more, 95% or more, 96% or more, 97% or more, 98% or more, or 99% or more, relative to the amino acid sequence of the (B1).

In the above (B1), the β2m protein derived from various animals may be, for example, a soluble β2m protein derived from various animals.

A conservative amino acid substitution may be introduced into the β2m protein, for example. The conservative amino acid substitution means, for example, a substitution by an amino acid residue having a side chain of properties similar to those of an amino acid to be substituted.

The β2m protein may be, for example, a polypeptide formed from a partial sequence of a β2m protein. In this case, a polypeptide formed from the partial sequence can form the complex when causing the polypeptide to coexist with a MR1 protein and ligand for a MAIT cell.

In the present invention, the β2m protein comprises, for example, a natural amino acid, a non-natural amino acid and/or a modified natural amino acid, and preferably comprises a natural amino acid and/or a modified natural amino acid. The amino acid constituting the β2m protein may be, for example, an L body, a D body, or both, and is preferably an L body.

The β2m protein may be an unmodified protein or a modified protein. Examples of the modification include: addition of functional groups, such as acylation, acetylation, alkylation, amidation, biotinylation, formylation, γ-carboxylation, glutamination, glycosylation, glycylation, isoprenylation, phosphorylation, PEGylation, racemization, and the like; and intraprotein crosslinking such as formation of disulfide bonds, and the like.

The method for producing each of the proteins is not particularly limited, and examples thereof include a chemical synthesis and a synthesis using the recombinant DNA technology. In the case of the chemical synthesis method, for example, each of the proteins can be produced by an organic synthesis method using a protective group, such as a benzyloxycarbonyl group (Cbz), a tert-butoxycarbonyl group (Boc), a fluorenylmethoxycarbonyl group (Fmoc), and the like. When the recombinant DNA technology is used, for example, each of the proteins can be produced by producing an expression vector including a polynucleotide encoding each protein, then producing an expression system of each of the proteins and producing each of the expressed proteins. The expression system can be produced, for example, by introducing the expression vector into a cell (host). Examples of the host include an animal cell, a plant cell, an insect cell, and a bacterial cell. Also, when the recombinant DNA technology is used, each of the proteins may be produced using, for example, a polynucleotide encoding each of the proteins and a cell-free translation system. Each of the proteins can be produced by isolating each of the proteins translated from the polynucleotide by the cell-free translation system.

The forming a complex forms a complex of the test substance, the MR1 protein, and the β2m protein by causing the test substance, the MR1 protein, and the β2m protein to coexist. The coexistence means, for example, that the test substance, the MR1 protein, and the β2m protein are present in the same reaction system, and as a specific example, the coexistence can be achieved by mixing the test substance, the MR1 protein, and the β2m protein in the presence of an aqueous solvent.

Examples of the aqueous solvent include: buffers such as tris hydroxymethylaminomethane (Tris), good buffers such as HEPES and the like; water such as pure water, distilled water; and the like. The aqueous solvent may include, for example, other components. Examples of the other components include: chelating agents, such as ethylenediaminetetraacetic acid (EDTA), ethylene glycol tetraacetic acid (EGTA), and the like; amino acids, such as glutamine, glycine, and the like; and denaturing agents, such as urea, guanidine salts, and the like.

In the forming a complex, the order of coexistence of the test substance, MR1 protein, and β2m protein is not particularly limited. For example, any two of them are caused to coexist first and the remaining one may be added thereto, or three of them may be mixed simultaneously. Preferably the MR1 protein and the β2m protein are caused to coexist first and then the test substance is mixed thereto.

In the forming a complex, for example, by reacting the obtained mixture after being caused to coexist, a complex of a candidate substance in the test substance, the MR1 protein, and the β2m protein can be formed. In the forming a complex, it is preferable to react the obtained mixture while stirring. The reaction time is, for example, 1 minute to 24 hours, 1 to 24 hours, or 3 to 12 hours. The reaction temperature is, for example, 0 to 50 °C, 0 to 37 °C, or 0 to 4 °C. The pH at the start of the reaction is, for example, 6 to 10, 7 to 9, or 7.5 to 8.5. As a specific example, the forming a complex is carried out for 3 hours or more at 0 to 10 °C and pH 7 to 9, for example. After the forming a complex, the capturing method of the present invention may, for example, dialyze the obtained complex. The dialysis can be carried out, for example, using a dialysis membrane. As the dialysate in the dialysis, for example, the aforementioned aqueous solvent can be used.

The test substance is not particularly limited, and examples thereof include a low molecular weight compound, a peptide, and a nucleic acid. The test substance may be a substance that forms the complex, a substance that does not form the complex, or a substance that is unknown to form or not form the complex. The test substance may comprise, for example, one kind of molecules or may comprise a plurality of kinds of molecules. In the latter case, the test substance may be, for example, a sample derived from a living body or a cell, or may be a library containing a plurality of kinds of homologous substances, such as a low molecular weight compound library, a peptide library, and the like. Examples of the bio-derived sample (biological sample) include a body fluid, a body fluid-derived cell, an organum, a tissue, an organ, and a cell separated from a living body. The body fluid may be, for example, blood, and specific examples thereof include whole blood, serum, and plasma. The body fluid-derived cell may be, for example, a blood-derived cell, and specific examples thereof include blood cells, such as a hemocyte, a leukocyte, a lymphocyte, and the like. Examples of the organ include digestive organs, such as a stomach, a pancreas, a colon, and a liver, a brain, and a peritoneum. Examples of the brain include a cerebrum, a temporal lobe, an occipital lobe, a cerebellum, a basal ganglion, and a mesenchymal tissue. Examples of the cell-derived sample include primary cultured cells and cultured cell lines. When the biological sample contains cells, the method preferably includes a step of crushing the cells prior to the forming a complex. The cell crushing can be performed by, for example, a method using a crushing apparatus such as a homogenizer, a method using an organic solvent such as phenol/chloroform, or the like. For example, the biological sample may be obtained by fractionating the obtained crushed product by chromatography or the like.

In the forming a complex, as a MR1 protein, for example, a cell expressing a MR1 protein (MR1 expressing cell), a MR1 protein held in a vehicle or a carrier (immobilized MR1 protein), or a soluble MR1 protein may be used, and a soluble MR1 protein is preferred. In the forming a complex, when the MR1 expressing cell is used as a MR1 protein, the MR1 expressing cell may be a cell that endogenously expresses a MR1 protein, or a cell that exogenously expresses a MR1 protein. The cell endogenously expressing the MR1 protein is not particularly limited, and examples thereof include: antigen-presenting cells, such as dendritic cells, macrophages, and the like; and double positive cells of the thymus. The cell exogenously expressing the MR1 protein can be prepared, for example, by introducing a polynucleotide or a vector encoding a MR1 protein into the cell. The vehicle includes, for example, membrane vesicles comprising lipids such as liposomes. The carrier may be, for example, beads and the like.

In the forming a complex, for example, as the β2m protein, a cell expressing a β2m protein (β2m expressing cell), a β2m protein held in a vehicle, a carrier, or the like, or a soluble β2m protein may be used, and a soluble β2m protein is preferred. In the forming a complex, when the β2m expressing cell is used as the β2m protein, a cell which endogenously expresses the β2m protein or a cell which exogenously expresses the β2m protein may be used as the β2m expressing cell. The cell which endogenously expresses the β2m protein is not particularly limited, and examples thereof include antigen presenting cells such as dendritic cells, macrophages, and the like. The cell exogenously expressing the β2m protein can be prepared, for example, by introducing a polynucleotide or a vector encoding the β2m protein into the cell.

When the MR1 expressing cell is used as the MR1 protein, in the forming a complex, it is preferable to use a MR1 expressing cell which expresses a β2m protein or to cause a MR1 expressing cell and a soluble β2m protein to coexist to use. When the β2m expressing cell is used as the β2m protein, in the forming a complex, it is preferable to use a β2m expressing cell which expresses a MR1 protein or to cause a β2m expressing cell and a soluble MR1 protein to coexist to use.

When the immobilized MR1 protein is used as the MR1 protein, in the forming a complex, it is preferable to use an immobilized MR1 protein in which a β2m protein is held or to cause the immobilized MR1 protein and a soluble β2m protein to coexist to use. When the immobilized β2m protein is used as the β2m protein, it is preferable to use an immobilized β2m protein in which a MR1 protein is held or to cause the immobilized β2m protein and a soluble MR1 protein to coexist to use.

Next, in the reducing, the complex is reduced with a reducing agent. The reducing can be carried out, for example, by causing the complex and the reducing agent to coexist, and specifically, by mixing the complex and the reducing agent in the presence of an aqueous solvent. Thus, in the reducing, the complex is reduced, and thereby, for example, a candidate substance can be immobilized to the complex in a state of being retained, so that the candidate substance can be prevented from falling off.

In the reducing, for example, by reacting the obtained mixture after being caused to coexist, the complex can be reduced. The reaction time is, for example, 1 minute to 4 hours, 0.5 to 4 hours, or 1 to 3 hours. The reaction temperature is, for example, 0 to 50°C, 0 to 37°C, or 0 to 10°C. The pH at the start of the reaction is, for example, 5 to 9, 6 to 8, or 6.5 to 7.5. As a specific example, when 25 to 75 mmol/l of sodium cyanoborohydride is used, the reaction temperature is 0 to 10°C, and the pH is 6.5 to 7.5.

In the complex, it is presumed that the candidate substance forms an imine (R-C(=NR')-CH₃, R': MR1 or β2m, R: a candidate substance) with the complex to bind. In addition, it is presumed that compared with the known ligand, a candidate substance falling out of the complex has a faster rate of hydrolysis of the imine, thereby more likely falling out of the complex. Therefore, in the reducing, it is preferable to reduce the imines in the complex to aminate (R-CH(-NHR')-CH₃, R': MR1 or β2m, R: a candidate substance). In this case, the reducing may also be referred to as, for example, a reductive aminating. The imines can also be referred to as, for example, a Schiff group or a Schiff base.

In addition, in the complex, it is presumed that a lysine residue of the MR1 protein and the test substance form an imine. Thus, in the reducing, it is more preferable that the imine formed from the lysine residue of the MR1 protein and the test substance is reduced and aminated. The lysine residue of the MR1 protein may be any lysine residue. The lysine residue of the MR1 protein is preferably a lysine residue to be mutated. Specifically, in the hMR1A, a lysine residue of the MR1 protein is, for example, a 43rd lysine residue in an extracellular domain.

As the reducing agent, for example, a reducing agent capable of reducing the imine can be used, and specifically, a hydride reducing agent used for hydride reduction can be used, for example. Examples of the hydride reducing agent include a metal hydride and a hydride complex, and a hydride complex is preferred. The metal hydride may be, for example, diboran (B₂H₆) or the like. Examples of the hydride complex include sodium borohydride (NaBH₄), sodium cyanoborohydride (NaBH₃CN), sodium triacetoxyborohydride (NaBH(OAc)₃), lithium triethylborohydride (LiBH(C₂H₅)₃), lithium aluminum hydride (LiAlH₄), diisobutylaluminum hydride ((i-B_{U})₂AlH), and lithium borohydride (LiBH₄), and sodium cyanoborohydride and sodium triacetoxyborohydride are preferred.

In this way, the capturing method of the present invention is capable of capturing a candidate substance in the complex.

It is preferable that the capturing method of the present invention includes a step of purifying the complex after the forming a complex and prior to the reducing. In this case, in the reducing, the complex obtained in the purifying is reduced with a reducing agent. Since the capturing method of the present invention can decrease in the MR1 protein and the β2m protein not forming a complex by including the purifying, the complex can be efficiently reduced in the reducing, and thus, for example, the candidate substance can be captured more efficiently. Thus, according to the capturing method including the purifying, a candidate substance that can bind to the complex can be captured more widely.

In the purifying, purification of the complex can be performed, for example, by a separation method for separating a monomer and a multimer of a protein, and specific examples thereof include: a separation method using an ion exchange column such as an anion exchange column or a cation exchange column; and gel filtration chromatography. Preferably, the anion exchange column is a column using a strong anion exchange resin. In the purifying, purification may be performed a plurality of times. When the purifying is performed using the anion exchange column, reference can be made to Example 1 described below. As a specific example, when the purifying is performed using the anion exchange column, the purification conditions may be, for example, the following purification conditions. When the purifying is performed by the gel filtration chromatography, reference can be made to, for example, Example 1 described below.

### (Purification condition)

Column: POROS^{™} HQ (manufactured by Applied Biosystems)
Starting buffer: 10 mmol/l Tris-HCl (pH 8.1)
Elution buffer: 10 mmol/l Tris-HCl (pH 8.1), 1 mol/l NaCl

### <Candidate substance producing method>

The present invention provides a method that can screen a candidate substance. As described above, the method for producing a candidate substance that can bind to a complex of a MR1 protein and a β2 microglobulin protein of the present invention includes steps of: forming a reduced complex of a reduced test substance, a MR1 protein, and a β2 microglobulin protein obtained by reducing a test substance, the MR1 protein, and the β2 microglobulin protein: and detecting the test substance in the reduced complex, wherein the forming the reduced complex is carried out by the method for capturing the candidate substance according to the present invention. The candidate substance producing method of the present invention is characterized in that the forming a reduced complex is carried out by the method for capturing a candidate substance of the present invention, and other steps and conditions are not particularly limited. According to the candidate substance producing method of the present invention, a candidate substance that can bind to the complex can be produced more widely. Regarding the candidate substance producing method of the present invention, reference can be made to the description of the capturing method of the present invention described above. The candidate substance producing method of the present invention can also be referred to as, for example, a method for screening a candidate substance of the present invention.

First, in the candidate substance producing method of the present invention, the forming a reduced complex is performed in the same manner as in the capturing method of the present invention.

Next, in the detecting, a test substance (candidate substance) in the reduced complex is detected. In the detecting, the test substance is detected directly or indirectly. The direct detection is the detection of the test substance. The indirect detection is detection of the test substance and the complex or a portion thereof as a unit. In the detection, for example, the structure of the test substance may be determined. In this case, the detecting may also be referred to as, for example, a step of analyzing or determining a structure.

In the direct detection, for example, the reduced complex is oxidized with an oxidizing agent, and the test substance is eluted from the obtained complex. Then, the eluted test substance is detected. The detection of the test substance can be performed, for example, by chromatography, such as liquid chromatography; nuclear magnetic resonance (NMR); mass spectrometry such as LC-MS, LC-MS/MS, GC-MS, or the like; and the like.

In the indirect detection, the complex or a portion thereof, and the test substance are detected as a unit. When detecting the complex and the test substance as a unit, the detection of the test substance can be performed, for example, by chromatography, such as liquid chromatography; nuclear magnetic resonance (NMR); mass spectrometry using LC-MS, LC-MS/MS, GC-MS, or the like; and the like. In the indirect detection, the detection, analysis, or structure determination of the test substance can be performed by comparing the detected result with the result of the detection of the complex.

When a portion of the complex and the test substance are detected as a unit, in the detecting, for example, the reduced complex is fragmented. The fragmentation can be performed, for example, by contacting the complex with a proteolytic enzyme or peptidase, or the like. Next, in the obtained fragment, the test substance is detected by detecting a peptide fragment containing the test substance. It is preferable that the peptide fragment containing the test substance contains a lysine residue of a MR1 protein-derived peptide to which the test substance is bound. The lysine residue of the MR1 protein-derived peptide is, for example, a lysine to be mutated. The detection of the test substance can be performed, for example, by chromatography, such as liquid chromatography; nuclear magnetic resonance (NMR); mass spectrometry using LC-MS, LC-MS/MS, GC-MS, or the like; and the like. Then, in the indirect detection, the detection, analysis, or structure determination of the test substance can be performed by comparing the detected result with the result of the detection of the complex.

In this way, the candidate substance producing method of the present invention can produce a candidate substance.

### <Ligand candidate substance producing method>

The present invention provides a method that can screen a ligand candidate for a MAIT cell. As described above, the method for producing a ligand candidate substance for a MAIT cell of the present invention includes steps of: detecting a candidate substance that binds to a complex of a MR1 protein and a β2 microglobulin protein from a test substance; contacting the candidate substance detected in the detecting, the MR1 protein, and the β2 microglobulin protein with a MAIT cell; and selecting a ligand candidate substance for a MAIT cell based on activation of the MAIT cell in the contacting, wherein the detecting is carried out by the candidate substance producing method according to the present invention. The ligand candidate substance producing method of the present invention is characterized in that the detecting is performed by the candidate substance producing method of the present invention, and other steps and conditions are not particularly limited. The ligand candidate substance producing method of the present invention can produce a ligand candidate substance for a MAIT cell more widely because, in the detecting, a candidate substance that can bind to the complex can be produced more widely. Regarding the ligand candidate substance producing method of the present invention, reference can be made to the descriptions of the capturing method and the candidate substance producing method of the present invention described above. The ligand candidate substance producing method of the present invention can also be referred to as, for example, a method for screening a ligand candidate substance of the present invention.

In the ligand candidate substance producing method of the present invention, the ligand candidate substance may be, for example, an agonist, a partial agonist, an antagonist, or an inverse agonist of the MAIT cell.

First, in the ligand candidate substance producing method of the present invention, the detecting is performed in the same manner as in the candidate substance producing method of the present invention.

Next, in the contacting, the candidate substance detected in the detecting, a MR1 protein, and a β2m protein are contacted with a MAIT cell. Specifically, in the contacting, a complex formed of the candidate substance detected in the detecting, the MR1 protein, and the β2m protein is contacted with the MAIT cell. The contacting can be performed, for example, in the presence of a medium. The medium is, for example, a medium for the MAIT cell. The medium may be preferably a medium free of a ligand for the MAIT cell such as folic acid, a metabolic source, and the like. Specifically, the medium may be, for example, a folic acid-free RPMI medium and the like. After the contacting, the obtained mixture is reacted. The reaction time is, for example, 1 to 48 hours, 6 to 48 hours, or 12 to 48 hours. The reaction temperature is, for example, 35 to 40°C or 36 to 38°C. The medium may contain, for example, a serum and a culture aid. Examples of the culture aid include (poly)amino acids, such as glutamine (L-glutamine), polyglutamine, a non-essential amino acid, and the like; and buffers such as sodium bicarbonate, and the like.

When a cell expressing the MR1 protein and the β2m protein (APC) is used in the contacting, a ratio (N_{M}:N_{A}) of a cell number of the MAIT cell (N_{M}) to a cell number of the APC (N_{A}) is, for example, 1:0.1 to 10, 1:1 to 10, or 1:2 to 5.

Among the MAIT cells, a human MAIT cell is, for example, a cell capable of recognizing 5-(2-oxopropylideneamino)-6-D-ribitylaminouracil(5-OP-RU) identified by human TRAV1-2 (T Cell Receptor Alpha Variable 1-2) with human TRBV6 or with human TRBV20-1 (T Cell Receptor Beta Variable 20-1) and displayed on the MR1 protein and the β2m protein. Among the MAIT cells, the mouse MAIT cell is, for example, a cell capable of recognizing a 5-OP-RU identified by mouse TRAV1 (T Cell Receptor Alpha Variable 1) with TRBV13 (T Cell Receptor Beta Variable 13) or with TRBV19 (T Cell Receptor Beta Variable 19) and displayed on the MR1 protein and the β2m protein. The MAIT cell may be obtained, for example, from intestinal tissues, intestinal lymph nodes, peripheral blood, or the like, or may be derived from stem cells or progenitor cells.

In the contacting, regarding the MR1 protein and the β2m protein, for example, reference can be made to the examples of the MR1 protein and the β2m protein described in the forming a complex. As a specific example, the MR1 protein and the β2m protein may be, for example, a protein expressed in a cell, a protein held in a vesicle or a carrier, or a soluble protein. When the MR1 protein and the β2m protein are proteins expressed in a cell, the complex formed by the candidate substance detected in the detecting, the MR1 protein, and the β2m protein is formed on the cell.

Next, in the selecting, a ligand candidate substance of the MAIT cell is selected based on the activation of the MAIT cell in the contacting. The activation of the MAIT cell may be, for example, the presence or absence of the activation of the MAIT cell or the degree of the activation of the MAIT cell. The activation of the MAIT cell may be, for example, activation of non-activated MAIT cells, i.e., steady-state MAIT cells, or suppression of the activation of activated MAIT cells. The activation of the MAIT cell can be evaluated by, for example, proliferation of MAIT cells; expression of cell-surface markers such as CD69 and the like; production of cytokines such as IFN-γ, TNF-α, and the like; production of chemokines such as MIP-1β, and the like; and the like. Then, in the selecting, for example, when the MAIT cell is activated, when the activation of the MAIT cell is suppressed, or when the activity of the MAIT cell is suppressed, as compared with a control to which no candidate substance detected in the detecting is added, the candidate substance can be selected as a ligand candidate substance of the MAIT cell.

In the ligand candidate substance producing method of the present invention, when an agonist or a partial agonist is selected, it is preferable to contact the candidate substance detected in the detecting, the MR1 protein, and the β2m protein, with the MAIT cell in the contacting, and a candidate substance that activates the MAIT cell in the contacting is selected as a ligand candidate substance of the MAIT cell in the selecting.

In addition, in the ligand candidate substance producing method of the present invention, when an antagonist is selected, it is preferable to contact the candidate substance detected in the detecting, the activator of the MAIT cell, the MR1 protein, and the β2m protein, with the MAIT cell in the contacting, and a candidate substance that suppresses the activation of the MAIT cell is selected as a ligand candidate substance for the MAIT cell in the selecting. As an activator of MAIT cell, for example, vitamin B9 (folic acid, vitamin M), diclofenac (2-(2-(2-(6-dichlorophenylamino)phenyl)acetic acid)), or the like can be used.

### <Capturing kit>

As described above, the kit for capturing a candidate substance that can bind to a complex of a MR1 protein and a β2 microglobulin protein of the present invention includes a MR1 protein; a β2 microglobulin protein; and a reducing agent, wherein the kit is used in the capturing method according to the present invention. The capturing kit of the present invention is characterized in that a MR1 protein, a β2 microglobulin protein, and a reducing agent are used in the capturing method of the present invention, and other configurations and conditions are not particularly limited. According to the capturing kit of the present invention, it is possible to capture a candidate substance that can bind to a complex of a MR1 protein and a β2m protein more widely. Regarding the capturing kit of the present invention, reference can be made to the descriptions of the capturing method, the candidate substance producing method, and the ligand candidate substance producing method of the present invention.

The capturing kit of the present invention can also be referred to as, for example, a recovery kit, a collection kit, or an extraction kit due to the ability to recover, collect, or extract a candidate substance that can bind to the complex from a test substance.

The MR1 protein, the β2m protein, and the reducing agent may each be contained in a separate container or may be contained in the same container in a mixed or unmixed manner. When the MR1 protein, the β2m protein, and the reducing agent are mixed and stored in the same container, the capturing kit of the present invention can also be referred to as a capture reagent.

The capturing kit of the present invention may include, for example, other components in addition to the MR1 protein, the β2m protein, and the reducing agent. The component may be, for example, instructions for use and the like.

Hereinafter, the present invention will be described in detail with reference to examples. The present invention, however, is not limited thereto.

### Examples

### Example 1

It was examined that a candidate substance that can bind to the complex can be captured more widely by the capturing method of the present invention.

When a MR1 protein, a β2 protein, and Ac6FP form a complex to form a normal folding, tryptophan in the protein can be measured at an absorbance of 330 nm. On the other hand, when the complex is heat treated and the complex is denatured, tryptophan in the protein is exposed by the thermal denaturation, and the tryptophan can be measured at an absorbance of 350 nm. Hence, it was examined whether or not the thermodynamic stability of the complex was improved by subjeting the complex to a reduction treatment.

### (1) Preparation of recombinant protein

A soluble mMR1A formed from the amino acid sequence of SEQ ID NO: 10 and a soluble mβ2m formed from the amino acid sequence of SEQ ID NO: 14 were prepared by the following procedure. First, an expression vector encoding each protein was prepared by a conventional method. The expression vectors were then introduced into Escherichia coli and cultured using 21 flasks to express each protein in Escherichia coli. The volume of the medium in the flask was 500 ml. After the culture, Escherichia coli was collected by centrifugation at 4000 rpm for 20 minutes at 4°C using a centrifuge (HP-25I, manufactured by Beckman Corporation). 20 ml of lysis buffer was added to the obtained pellet. The composition of the lysis buffer was as follows: 10 mmol/l Tris-HCl, 10 mmol/l MgCl₂, 150 mmol/l NaCl, and 10% Glycerol.

The obtained samples were crushed on ice using an ultrasonic homogenizer (UD-211, manufactured by Tomy Co., Ltd.). The conditions for the sonication were as follows: Output: 6, Duty: 5, and 10 minutes. After the crushing, 100 µl of 20 mg/ml DNase was added to the obtained crushing solution (final concentration: 0.1 mg/ml). The crushing solution after the addition was shaken at room temperature (about 25 °C) for 30 minutes.

Next, 50 ml of a washing solution was added to the crushing solution. The composition of the washing solution was as follows: 0.5% Triton X-100, 50 mmol/l Tris-HCl, 100 mmol/l NaCl, and 10 mmol/l EDTA. After the addition, the obtained mixed solution was centrifuged at 8000 rpm for 20 minutes at 4°C using the centrifuge. The obtained pellet was suspended with 50 ml of the washing solution and then centrifuged at 8000 rpm for 20 minutes at 4°C using the centrifuge. 50 ml of a resuspension buffer was added to the obtained pellet. The composition of the resuspension buffer was as follows: 50 mmol/l Tris-HCl, 100 mmol/l NaCl, and 10 mmol/l EDTA. Then, the obtained suspension was centrifuged at 8000 rpm for 20 minutes at 4°C. The obtained pellet was suspended with 50 ml of the washing solution and then centrifuged at 8000 rpm for 20 minutes at 4°C using a centrifuge.

To the obtained pellet, a minimal amount (about 5 to 10 ml) of a guanidine buffer in which the pellet is to be dissolved was added, and the pellet was dissolved. The composition of the guanidine buffer was as follows: 6 mol/l Guanidine, 50 mmol/l Tris-HCl, 100 mmol/l NaCl, and 2 mmol/l EDTA. When the pellet was not completely dissolved after suspension, the resultant was incubated at 37°C for 1 hour. The obtained lysate was centrifuged at 8000 rpm for 20 minutes at 4°C using the centrifuge. After the centrifugation, the supernatant was recovered and stored at -30°C or -80°C until use.

### (2) Complex forming

After mixing the soluble mMR1A and the soluble mβ2m obtained in Example 1 (1), Ac6FP (Cat.No.: 11.418, manufactured by Schircks Laboratories Co., Ltd.) was further added to the obtained mixed solution to form the complex in a buffer solution (reaction system of complex). The composition of the buffer was as follows: 100 mmol/l Tris-HCl (pH 8.1), 2 mmol/l EDTA (pH 8.1), 400 mmol/l L-Arginine, and 5 mol/l Urea, and the pH was set to 8.1. In the reaction system of 250 ml of complex, the concentration of the soluble mMR1A was 30 µg/ml (total amount: 7.5 mg), the concentration of the soluble mβ2m was 30 µg/ml (total amount: 7.5 mg), and the concentration of Ac6FP was 8 µg/ml (total amount: 2 mg). The reaction temperature of the reaction system of the complex was set at 4°C, and the reaction time was set at 9 hours.

### (3) Purification of complex

After forming the complex, the obtained mixed solution was introduced into a dialysis membrane (Dialysis tubing cellulose, Cat.No.:D9402-100FT, manufactured by SIGMA-ALDRICH Co., Ltd.) and dialyzed in a dialysate at 4°C for 36 hours. After the dialysis, the dialysate was replaced and further dialyzed for 36 hours at 4°C. The dialysate was set to 10 mmol/l Tris-HCl (pH 8.1).

After the dialysis, the obtained sample was subjected to anion exchange chromatography under the purification condition 1 below to purify a crude purified product including the complex. After the start of the purification, the resultant was fractionated at 1ml/fraction, and five fractions between 6 to 7 minutes from the start of purification were collected as fractions of the crude purified product.

### (Purification Condition 1)

Analytical instrument: AKTA pure25 (manufactured by GE Healthcare)
Column: POROS^{™} HQ (manufactured by Applied Biosystems)
   (Inner diameter: 10 mm × 10 cm)
Starting Buffer (Solvent A): 10 mmol/l Tris-HCl (pH8.1)
Elution buffer (Solvent B): 10 mmol/l Tris-HCl (pH8.1), 1 mol/l NaCl
Elution conditions: the proportion of Solvent A was linearly decreased and the proportion of Solvent B was linearly increased from 0 to 7.9 minutes, and elution was performed so as to achieve the followings:
   time: (solvent ratio (A:B))
   0 min: (A:B = 100:0)
   7.9 min: (A:B = 50:50)
Flow rate: 5 ml/min
Fractionation volume: 1 ml/fraction

The obtained composition was then subjected to gel filtration chromatography under the purification condition 2 below to purify the complex from the crude purified product. After the start of the purification, the resultant was fractionated at 0.5ml/fraction, and 4 fractions between 13 and 15 minutes from the start of purification were collected as a fraction containing the complex.

### (Purification Condition 2)

Detection device: AKTA pure25 (manufactured by GE Healthcare)
Column: Superdex 200 Increase 10/300 GL (manufactured by GE Healthcare)
   (Inner diameter: 10 mm × 30 cm)
Gel filtration resin: composite of cross-linked agarose and dextran (manufactured by GE Healthcare)
Column equilibration buffer: 10 mmol/l Tris-HCl (pH8.1), 10 mmol/l NaCl
Mobile phase:
   Solvent: 10 mmol/l Tris-HCl (pH 8.1), 10 mmol/l NaCl
   Flow rate: 0.5 ml/min
   Fractionation volume: 0.5 ml/fraction

### (4) Reduction of the complex

Sodium cyanoborohydride (NaBH₃CN) was added to the obtained complex (0.01 mg/ml), so as to have a predetermined concentration (0, 0.005, 0.05, 0.5, or 5 mmol/l) and subjected to the reduction treatment at 4 °C for 5 minutes. Note that the pH in the reaction solution of the reduction reaction was set to about 7.2.

### (5) Stability of complex

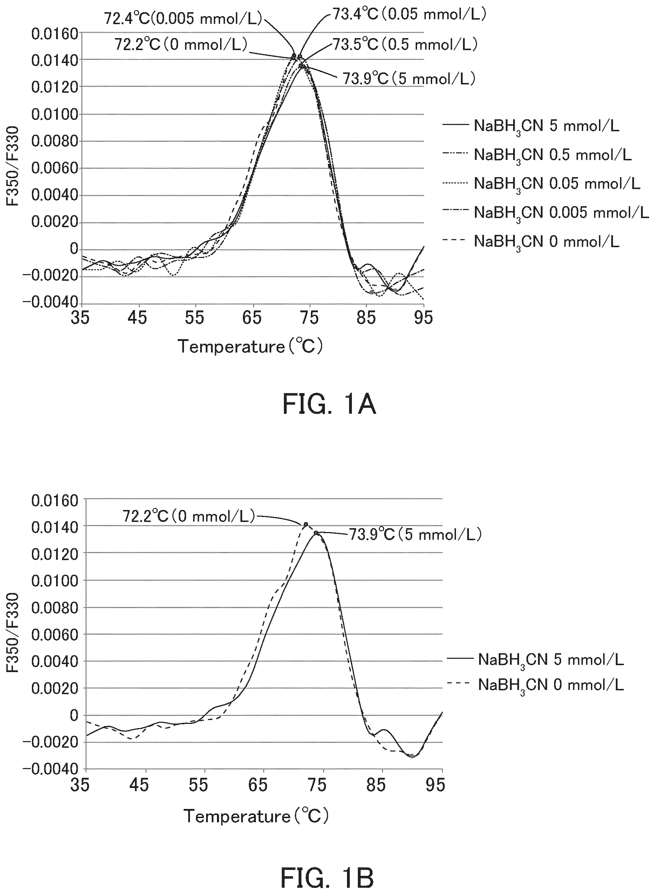

8 to 10 µl of a sample containing the complex after the reduction treatment was collected in a capillary and set to Tycho^{™} NT.6. The temperature was then increased to 37 to 95°C, and the thermodynamic stability of the complex was calculated from the melting point (an inflection point of curve). These results are shown in FIGs. 1A and 1B.

FIGs. 1A and 1B are graphs of the thermodynamic stability of the complex. In FIGs. 1A and 1B, the horizontal axis indicates the temperature, and the vertical axis indicates the ratio of the absorbance at 350 nm (F350) to the absorbance at 330 nm (F330) (F350/F330). As shown in FIG. 1A, due to the reduction treatment, the melting point was increased as compared to the control not subjected to the reduction treatment. Further, as shown in FIG. 1B, the melting point of the control not subjected to the reduction treatment was 72.2°C, whereas the melting point of the complex treated with 5 mmol/l of sodium cyanoborohydride was 73.9°C, which is higher than the control. From these results, it was found that the thermodynamic stability of the complex was improved by the reduction treatment. In addition, since the thermodynamic stability of the complex is improved by the reduction treatment, candidate substances, which do not form a complex with the MR1 protein and the β2m protein and have low stability when the reduction treatment is not performed, can be retained in a state of forming a complex with the MR1 protein and the β2m protein. Therefore, according to the capturing method of the present invention, a candidate substance that can bind to a complex can be captured more widely.

### Example 2

It was examined that a candidate substance that can bind to the complex can be captured more widely by the capturing method of the present invention.

### (1) Preparation of recombinant protein

A soluble mMR1A formed from the amino acid sequence of SEQ ID NO: 10 and a soluble hβ2m formed from the amino acid sequence of SEQ ID NO: 13 were prepared in the same manner as in Example 1 (1), except that an expression vector encoding a soluble hβ2m was prepared and used, instead of the expression vector encoding the soluble mβ2m.

### (2) Complex forming

After mixing the soluble mMR1A and the soluble hβ2m obtained in Example 2(1), 3 mg of a component derived from a pancreas of a wild-type mouse (C57BL/6, purchased from Nippon Clare) was further added to the obtained mixed solution, and the complex was formed in a buffer solution (a reaction system of the complex). The composition of the buffer was as follows: 100 mmol/l Tris-HCl (pH 8.1), 2 mmol/l EDTA (pH 8.1), 400 mmol/l L-Arginine, and 5 mol/l Urea, and the pH was 8.1. In the reaction system of 250 ml of the complex, the concentration of the soluble mMR1A was 30 µg/ml (total amount: 7.5 mg), and the concentration of the soluble hβ2m was 30 µg/ml (total amount: 7.5 mg). The reaction temperature of the reaction system of the complex was set to 4°C, and the reaction time was set to 9 hours. The components derived from the pancreas were prepared by the following procedure. First, the pancreas of the wild-type mouse was homogenized using an ultrasonic generator (UR-21P, manufactured by TOMY Corporation). The obtained treatment solution was centrifuged at 15,000×g for 15 minutes, and then the supernatant fraction thereof was collected. The supernatant fraction was subjected to liquid chromatography under the purification condition 3 below to purify a fraction assumed to contain a molecule that binds to mMR1 and hβ2m. After the start of the purification, the resultant was fractionated at 0.5 ml/fraction, and 1 fraction (29th fraction (Fr29)) between 14 and 16 minutes from the start of the purification was collected as a fraction containing a component derived from the pancreas. The Fr29 was dried with nitrogen gas, and the weight was measured with a microbalance, threby quantifying the amount of the compound contained in the Fr29.

### (Purification condition 3)

Analytical instrument: AKTA pure25 (manufactured by GE Healthcare)
Column: COSMOSIL 5C₁₈-MS-II Packed Column (manufactured by Nacalai Tesque, Inc.)
   (Inner diameter: 4.6 mm×250 mm)
Solvent A: 0.1 (v/v)% trifluoroacetic acid aqueous solution
Solvent B: 0.1 (v/v)% trifluoroacetic acid-80 (v/v)% acetonitrile aqueous solution Elution conditions: The proportion of Solvent A was linearly decreased and the proportion of Solvent B was linearly increased from 7 to 57 minutes, and elution was performed so as to achieve the followings:
   time: (solvent ratio (A:B))
   0 min: (A:B = 100:0)
   7 minutes: (A:B = 100:0)
   57 minutes: (A:B = 0:100)
   62 minutes: (A:B = 0:100)
Flow rate: 1 ml/min
Fractionation volume: 0.5 ml/fraction

### (3) Purification of complex

A sample comprising the complex was subjected to anion exchange chromatography under the purification condition 4 below to purify a crude purified product containing the complex. After the start of the purification, the resultant was fractionated at 1 ml/fraction, and two fractions (32nd fraction (Fr32) and 33rd fraction (Fr33)) between 6 and 7 minutes from the start of purification were collected as fractions of purified products.

### (Purification Condition 4)

Analytical instrument: AKTA pure25 (manufactured by GE Healthcare)
Column: POROS^{™} HQ (manufactured by Applied Biosystems)
   (Inner diameter: 10 mm × 10 cm)
Starting Buffer (Solvent A): 10 mmol/l Tris-HCl (pH8.1)
Elution buffer (Solvent B): 10 mmol/l Tris-HCl (pH8.1), 1 mol/l NaCl
Elution conditions: The proportion of Solvent A was linearly decreased and the proportion of Solvent B was linearly increased from 0 to 7.9 minutes, and elution was performed so as to achieve the followings:
   time: (solvent ratio (A:B))
   0 min: (A:B = 100:0)
   7.9 min: (A:B = 50:50)
Flow rate: 5 ml/min
Fractionation volume: 1 ml/fraction

### (4) Reduction of complex

Sodium cyanoborohydride (NaBH₃CN) was added to the obtained complex (0.01 mg/ml) so as to achieve 50 mmol/l, and the resultant was subjected to the reduction treatment at 4°C for 5 minutes. Note that the pH in the reaction solution of the reduction reaction was set to about 7.2.

### (5) Fragmentation of complex

After the reduction, a surfactant (ProteaseMAX^{™} Surfactant, manufacutured by Promega Co., Ltd.) was added to the obtained reaction solution so that the final concentration became 0.1 (v/v)%. Note that the surfactant, which was dissolved in 1 mol/l guanidine-HCl and 100 mmol/l Tris-HCl (pH 7.5), was used. Next, dithiothreitol (DTT) was added to the reaction solution so that the final concentration became 5 mmol/l, and then the reaction solution was incubated at 50 to 60°C for 20 minutes. After the incubation, the reaction solution was cooled to the room temperature.

After the cooling, iodoacetamide was added to the reaction solution so that the final concentration became 15 mmol/l, followed by incubation under dark conditions at the room temperature for 15 minutes, thereby being alkylated. After the incubation, a buffer (pH 7.5) was added to the reaction solution, the reaction solution was adjusted to have a protein concentration for use in peptide digestion and was used as a sample solution to be subjected to the peptide digestion. The composition of the buffer was 1 mol/l Guanidine HCl.

Protease (Asp-N, Sequencing Grade, manufactured by Promega) was added to the sample solution so that a weight ratio (P_{A}:P_{P}) of protease (P_{A}) to protein (P_{P}) in the sample solution became 1:20, and a mixed solution was prepared. After the addition, the mixed solution was stirred using a vortex mixer, and then centrifuged in a tabletop centrifuge and spun down. The mixed solution was then incubated for 18 hours at 37°C to degrade the protein.

### (6) Detection of candidate substance

Using a liquid chromatograph system (EASY-nLC 1200 UHPLC, manufactured by Thermo Fisher Scientific Co., Ltd.) and a mass spectrometer (Q Exactive Plus, manufactured by Thermo Fisher Scientific Co., Ltd.), the sample solution after the proteolysis was measured under the conditions of the mass spectrometry method described below. The control of the mass spectrometer and the collection of mass data were performed using the attached software. Except that Ac6FP (concentration of Ac6FP in the reaction system of the complex: 8 µg/ml (total amount: 2 mg)) was used as a positive control, instead of the pancreatic processed product of the wild-type mouse, and that the control was not treated with the reducing agent, the mass spectrometry was performed in the same manner. In each group, the same experiment was repeated using three types of samples (samples 1 to 3).

### (Conditions of mass spectrometry)

(1) Column: C18 reverse phase column (manufactured by Nikkyo Technos, Co., Ltd.)
   (Inner diameter 75 µm× length 150 mm)
(2) Mobile phase:
   The proportion of Solvent A was linearly decreased and the proportion of Solvent B was linearly increased from 0 to 100 minutes and thereafter the ratio between Solvent A and Solvent B was maintained at 20:80 for 10 minutes (from 100 to 110 minutes) so as to achieve the followings:
   Solvent A: 0.1 (v/v)% formic acid aqueous solution (manufactured by NACALAI TESQUE, INC.)
   Solvent B: 0.1 (v/v) % formic acid-acetonitrile (manufactured by NACALAI TESQUE, INC.)
      Gradient conditions: time (solvent ratio (A:B))
      0 min: (A:B = 96:4)
      100 minutes: (A:B = 72:28)
(3) Flow rate: 0.2 ml/min

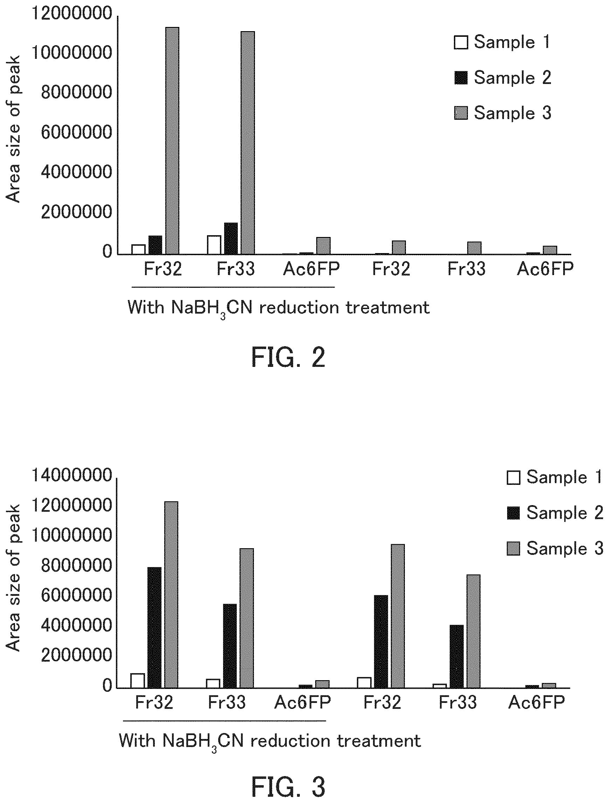

Regarding the obtained data, the area size of each peak in the m/z axis was obtained, and significantly different peaks depending on with or without the reduction treatment were detected. The results are shown in FIGs. 2 and 3. Note that since all the peaks are derived from peptide fragments containing the 43rd lysine in the extracellular domain, it was confirmed that the candidate substance captured in the complex of a MR1 and a β2m was bound to the 43rd lysine in the extracellular domain.

FIG. 2 is a graph showing the data having different (or no) peaks (m/z = 84.986) depending on with or without the reduction treatment. In FIG. 2, the horizontal axis indicates the type of the sample, and the vertical axis indicates the area size of the peak. As shown in FIG. 2, the peak of m/z = 84.986 was not observed when the reduction treatment was not performed and was observed only when the reduction treatment was performed. That is, a substance corresponding to a peak of m/z = 84.986 can be a candidate substance to be captured in a complex with a MR1 and a β2m obtained for the first time by undergoing the reduction treatment.

Next, FIG. 3 is a graph showing the data having different area sizes of peaks (m/z = 229.1350) depending on with or without the reduction treatment. In FIG. 3, the horizontal axis indicates the type of the sample, and the vertical axis indicates the area size of the peak. As shown in FIG. 3, when the reduction treatment was performed, the area size of the peak at m/z = 229.1350 was significantly increased as compared with the case where the reduction treatment was not performed. In other words, a substance corresponding to the peak of m/z = 229.1350 can be a candidate substance which is more stably captured in a complex formed by a MR1 and a β2m by undergoing a reduction treatment. In addition, in the peaks of substances other than those correspnding to the peak of m/z = 229.1350, a tendency of increase in an area size was similarly observed.

From the above, it was confirmed that, according to the capturing method of the present invention, a candidate substance that can bind to the complex can be captured more widely, and that the captured candidate substance can be held more stably in the complex.

While the present invention has been described above with reference to illustrative embodiments, the present invention is by no means limited thereto. Various changes and variations that may become apparent to those skilled in the art may be made in the configuration and specifics of the present invention without departing from the scope of the present invention.

This application claims priority from Japanese Patent Application No. 2019-211860 filed on November 22, 2019. The entire subject matter of the Japanese Patent Applications is incorporated herein by reference.

### (Supplementary Notes)

Some or all of the above embodiments and examples may be described as in the following Supplementary Notes, but are not limited thereto.

### (Supplementary Note 1)

A method for capturing a candidate substance that can bind to a complex of a MR1 protein and a β2 microglobulin protein, including steps of:
forming a complex of a test substance, the MR1 protein, and the β2 microglobulin protein by causing the test substance, the MR1 protein, and the β2 microglobulin protein to coexist; and
reducing the complex with a reducing agent.

### (Supplementary Note 2)

The method for capturing according to Supplementary Note 1, wherein
in the reducing, an imine in the complex is reduced and aminated.

### (Supplementary Note 3)

The method for capturing according to Supplementary Note 1 or 2, wherein
in the reducing, an imine formed by a lysine residue of the MR1 protein and the test substance is reduced and aminated.

### (Supplementary Note 4)

The method for capturing according to any one of Supplementary Notes 1 to 3, including a step of:
purifying the complex after being formed, wherein
in the reducing, the complex obtained in the purifying is reduced with the reducing agent.

### (Supplementary Note 5)

The method for capturing according to any one of Supplementary Notes 1 to 4, wherein
in the reducing, the complex is reduced with a hydride reducing agent.

### (Supplementary Note 6)

A method for producing a candidate substance that can bind to a complex of a MR1 protein and a β2 microglobulin protein, including steps of:
forming a reduced complex of a reduced test substance, the MR1 protein, and the β2 microglobulin protein, obtained by reducing a test substance, the MR1 protein, and the β2 microglobulin protein; and
detecting the test substance in the reduced complex, wherein
the forming the reduced complex is carried out by the method for capturing a candidate substance according to any one of Supplementary Notes 1 to 5.

### (Supplementary Note 7)

The method for producing a candidate substance according to Supplementary Note 6, wherein
in the detecting,
the reduced complex is fragmented, and
a peptide fragment including the test substance is detected.

### (Supplementary Note 8)

The method for producing according to Supplementary Note 7, wherein
the peptide fragment containing the test substance includes a lysine residue of a MR1 protein-derived peptide to which the test substance is bound.

### (Supplementary Note 9)

A method for producing a ligand candidate substance for a MAIT cell, including steps of:
detecting a candidate substance that binds to a complex of a MR1 protein and a β2 microglobulin protein from test substances;
contacting the candidate substance detected in the detecting, the MR1 protein, and the β2 microglobulin protein, with a mucosal-associated invariant T (MAIT) cell; and
selecting a ligand candidate substance for the MAIT cell based on activation of the MAIT cell in the contacting, wherein
the detecting is carried out by the method for producing a candidate substance according to any one of Supplementary Notes 6 to 8.

### (Supplementary Note 10)

The method for producing a ligand candidate substance according to Supplementary Note 9, wherein
in the contacting, the candidate substance detected in the detecting, the MR1 protein, and the β2 microglobulin protein are contacted with the MAIT cell, and
in the selecting, a candidate substance for activating the MAIT cell is selected as the ligand candidate substance for the MAIT cell.

### (Supplementary Note 11)

The method for producing a ligand candidate substance according to Supplementary Note 9, wherein
in the contacting, the candidate substance detected in the detecting, an activator of the MAIT cell, the MR1 protein, and the β2 microglobulin protein are contacted with the MAIT cell, and in the selecting, a candidate substance for suppressing activation of the MAIT cell is selected as the ligand candidate substance for the MAIT cell.

### (Supplementary Note 12)

A kit for capturing a candidate substance that can bind to a complex of a MR1 protein and a β2 microglobulin protein, including:
the MR1 protein;
the β2 microglobulin protein; and
the reducing agent, wherein
the kit is used in the method for capturing according to any one of Supplementary Notes 1 to 5.

### Industrial Applicability

As described above, according to the present invention, a candidate substance that can bind to the complex can be captured more widely. For this reason, according to the present invention, for example, a ligand other than a vitamin metabolite or a candidate substance thereof can be identified. Thus, the present invention is extremely useful, for example, in a research field, a pharmaceutical field, and the like.

## Claims

1. A method for capturing a candidate substance that can bind to a complex of a MR1 protein and a β2 microglobulin protein, comprising steps of:
forming a complex of a test substance, the MR1 protein, and the β2 microglobulin protein by causing the test substance, the MR1 protein, and the β2 microglobulin protein to coexist; and
reducing the complex with a reducing agent.

2. The method for capturing according to claim 1, wherein
in the reducing, an imine in the complex is reduced and aminated.

3. The method for capturing according to claim 1 or 2, wherein
in the reducing, an imine formed by a lysine residue of the MR1 protein and the test substance is reduced and aminated.

4. The method for capturing according to any one of claims 1 to 3, comprising a step of:
purifying the complex after being formed, wherein
in the reducing, the complex obtained in the purifying is reduced with the reducing agent.

5. The method for capturing according to any one of claims 1 to 4, wherein
in the reducing, the complex is reduced with a hydride reducing agent.

6. A method for producing a candidate substance that can bind to a complex of a MR1 protein and a β2 microglobulin protein, comprising steps of:
forming a reduced complex of a reduced test substance, the MR1 protein, and the β2 microglobulin protein obtained by reducing a test substance, the MR1 protein, and the β2 microglobulin protein; and
detecting the test substance in the reduced complex, wherein
the forming the reduced complex is carried out by the method for capturing a candidate substance according to any one of claims 1 to 5.

7. The method for producing a candidate substance according to claim 6, wherein
in the detecting,
the reduced complex is fragmented, and
a peptide fragment comprising the test substance is detected.

8. The method for producing according to claim 7, wherein
the peptide fragment comprising the test substance comprises a lysine residue of a MR1 protein-derived peptide to which the test substance is bound.

9. A method for producing a ligand candidate substance for a MAIT cell, comprising the steps of:
detecting a candidate substance that binds to a complex of a MR1 protein and a β2 microglobulin protein from test substances;
contacting the candidate substance detected in the detecting, the MR1 protein, and the β2 microglobulin protein with a mucosal-associated invariant T (MAIT) cell; and
selecting a ligand candidate substance for the MAIT cell based on activation of the MAIT cell in the contacting, wherein
the detecting is carried out by the method for producing a candidate substance according to any one of claims 6 to 8.

10. A kit for capturing a candidate substance that can bind to a complex of a MR1 protein and a β2 microglobulin protein, comprising:
the MR1 protein;
the β2 microglobulin protein; and
the reducing agent, wherein
the kit is used in the method for capturing according to any one of claims 1 to 5.


### [Sequence Listing]

TF19252WO_Sequence listing_ST25.txt

SEQUENCE LISTING

<110>  Osaka Univesity

<120>  Method of capturing a candidate substance that can bind to the
       complex of MR1 and beta2 microglobulin, method of producing a
       candidate substance that can bind to the complex of MR1 and beta2
        microglobulin, and method of producing a ligand candidate
       substance of MAIT cell

<130>  TF19252WO

<150>  JP2019-211860
<151>  2019-11-22

<160>  14

<170>  PatentIn version 3.5

<210>  1
<211>  341
<212>  PRT
<213>  Homo sapiens

<400>  1

Met Gly Glu Leu Met Ala Phe Leu Leu Pro Leu Ile Ile Val Leu Met
1               5                   10                  15

Val Lys His Ser Asp Ser Arg Thr His Ser Leu Arg Tyr Phe Arg Leu
            20                  25                  30

Gly Val Ser Asp Pro Ile His Gly Val Pro Glu Phe Ile Ser Val Gly
        35                  40                  45

Tyr Val Asp Ser His Pro Ile Thr Thr Tyr Asp Ser Val Thr Arg Gln
    50                  55                  60

Lys Glu Pro Arg Ala Pro Trp Met Ala Glu Asn Leu Ala Pro Asp His
65                  70                  75                  80

Trp Glu Arg Tyr Thr Gln Leu Leu Arg Gly Trp Gln Gln Met Phe Lys
                85                  90                  95

Val Glu Leu Lys Arg Leu Gln Arg His Tyr Asn His Ser Gly Ser His
            100                 105                 110

Thr Tyr Gln Arg Met Ile Gly Cys Glu Leu Leu Glu Asp Gly Ser Thr
        115                 120                 125

Thr Gly Phe Leu Gln Tyr Ala Tyr Asp Gly Gln Asp Phe Leu Ile Phe
    130                 135                 140

Asn Lys Asp Thr Leu Ser Trp Leu Ala Val Asp Asn Val Ala His Thr

145                 150                 155                 160

Ile Lys Gln Ala Trp Glu Ala Asn Gln His Glu Leu Leu Tyr Gln Lys
                165                 170                 175

Asn Trp Leu Glu Glu Glu Cys Ile Ala Trp Leu Lys Arg Phe Leu Glu
            180                 185                 190

Tyr Gly Lys Asp Thr Leu Gln Arg Thr Glu Pro Pro Leu Val Arg Val
        195                 200                 205

Asn Arg Lys Glu Thr Phe Pro Gly Val Thr Ala Leu Phe Cys Lys Ala
    210                 215                 220

His Gly Phe Tyr Pro Pro Glu Ile Tyr Met Thr Trp Met Lys Asn Gly
225                 230                 235                 240

Glu Glu Ile Val Gln Glu Ile Asp Tyr Gly Asp Ile Leu Pro Ser Gly
                245                 250                 255

Asp Gly Thr Tyr Gln Ala Trp Ala Ser Ile Glu Leu Asp Pro Gln Ser
            260                 265                 270

Ser Asn Leu Tyr Ser Cys His Val Glu His Cys Gly Val His Met Val
        275                 280                 285

Leu Gln Val Pro Gln Glu Ser Glu Thr Ile Pro Leu Val Met Lys Ala
    290                 295                 300

Val Ser Gly Ser Ile Val Leu Val Ile Val Leu Ala Gly Val Gly Val
305                 310                 315                 320

Leu Val Trp Arg Arg Arg Pro Arg Glu Gln Asn Gly Ala Ile Tyr Leu
                325                 330                 335

Pro Thr Pro Asp Arg
            340

<210>  2
<211>  249
<212>  PRT
<213>  Homo sapiens

<400>  2

Met Gly Glu Leu Met Ala Phe Leu Leu Pro Leu Ile Ile Val Leu Met
1               5                   10                  15

Val Lys His Ser Asp Ser Arg Thr His Ser Leu Arg Tyr Phe Arg Leu

20                  25                  30

Gly Val Ser Asp Pro Ile His Gly Val Pro Glu Phe Ile Ser Val Gly
        35                  40                  45

Tyr Val Asp Ser His Pro Ile Thr Thr Tyr Asp Ser Val Thr Arg Gln
    50                  55                  60

Lys Glu Pro Arg Ala Pro Trp Met Ala Glu Asn Leu Ala Pro Asp His
65                  70                  75                  80

Trp Glu Arg Tyr Thr Gln Leu Leu Arg Gly Trp Gln Gln Met Phe Lys
                85                  90                  95

Val Glu Leu Lys Arg Leu Gln Arg His Tyr Asn His Ser Gly Ser His
            100                 105                 110

Thr Tyr Gln Arg Met Ile Gly Cys Glu Leu Leu Glu Asp Gly Ser Thr
        115                 120                 125

Thr Gly Phe Leu Gln Tyr Ala Tyr Asp Gly Gln Asp Phe Leu Ile Phe
    130                 135                 140

Asn Lys Asp Thr Leu Ser Trp Leu Ala Val Asp Asn Val Ala His Thr
145                 150                 155                 160

Ile Lys Gln Ala Trp Glu Ala Asn Gln His Glu Leu Leu Tyr Gln Lys
                165                 170                 175

Asn Trp Leu Glu Glu Glu Cys Ile Ala Trp Leu Lys Arg Phe Leu Glu
            180                 185                 190

Tyr Gly Lys Asp Thr Leu Gln Arg Thr Glu Ser Glu Thr Ile Pro Leu
        195                 200                 205

Val Met Lys Ala Val Ser Gly Ser Ile Val Leu Val Ile Val Leu Ala
    210                 215                 220

Gly Val Gly Val Leu Val Trp Arg Arg Arg Pro Arg Glu Gln Asn Gly
225                 230                 235                 240

Ala Ile Tyr Leu Pro Thr Pro Asp Arg
                245

<210>  3
<211>  205
<212>  PRT
<213>  Homo sapiens

<400>  3

Met Val Lys His Ser Asp Ser Arg Thr His Ser Leu Arg Tyr Phe Arg
1               5                   10                  15

Leu Gly Val Ser Asp Pro Ile His Gly Val Pro Glu Phe Ile Ser Val
            20                  25                  30

Gly Tyr Val Asp Ser His Pro Ile Thr Thr Tyr Asp Ser Val Thr Arg
        35                  40                  45

Gln Lys Glu Pro Arg Ala Pro Trp Met Ala Glu Asn Leu Ala Pro Asp
    50                  55                  60

His Trp Glu Arg Tyr Thr Gln Leu Leu Arg Gly Trp Gln Gln Met Phe
65                  70                  75                  80

Lys Val Glu Leu Lys Arg Leu Gln Arg His Tyr Asn His Ser Gly Ser
                85                  90                  95

His Thr Tyr Gln Arg Met Ile Gly Cys Glu Leu Leu Glu Asp Gly Ser
            100                 105                 110

Thr Thr Gly Phe Leu Gln Tyr Ala Tyr Asp Gly Gln Asp Phe Leu Ile
        115                 120                 125

Phe Asn Lys Asp Thr Leu Ser Trp Leu Ala Val Asp Asn Val Ala His
    130                 135                 140

Thr Ile Lys Gln Ala Trp Glu Ala Asn Gln His Glu Leu Leu Tyr Gln
145                 150                 155                 160

Lys Asn Trp Leu Glu Glu Glu Cys Ile Ala Trp Leu Lys Arg Phe Leu
                165                 170                 175

Glu Tyr Gly Lys Asp Thr Leu Gln Arg Thr Gly Lys Glu Lys Glu Lys
            180                 185                 190

Ala Ser Phe Pro His Cys Leu Asn Asn Cys Phe Tyr Thr
        195                 200                 205

<210>  4
<211>  245
<212>  PRT
<213>  Homo sapiens

<400>  4

Met Ala Phe Leu Leu Pro Leu Ile Ile Val Leu Met Val Lys His Ser

1               5                   10                  15

Asp Ser Arg Thr His Ser Leu Arg Tyr Phe Arg Leu Gly Val Ser Asp
            20                  25                  30

Pro Ile His Gly Val Pro Glu Phe Ile Ser Val Gly Tyr Val Asp Ser
        35                  40                  45

His Pro Ile Thr Thr Tyr Asp Ser Val Thr Arg Gln Lys Glu Pro Arg
    50                  55                  60

Ala Pro Trp Met Ala Glu Asn Leu Ala Pro Asp His Trp Glu Arg Tyr
65                  70                  75                  80

Thr Gln Leu Leu Arg Gly Trp Gln Gln Met Phe Lys Val Glu Leu Lys
                85                  90                  95

Arg Leu Gln Arg His Tyr Asn His Ser Gly Ser His Thr Tyr Gln Arg
            100                 105                 110

Met Ile Gly Cys Glu Leu Leu Glu Asp Gly Ser Thr Thr Gly Phe Leu
        115                 120                 125

Gln Tyr Ala Tyr Asp Gly Gln Asp Phe Leu Ile Phe Asn Lys Asp Thr
    130                 135                 140

Leu Ser Trp Leu Ala Val Asp Asn Val Ala His Thr Ile Lys Gln Ala
145                 150                 155                 160

Trp Glu Ala Asn Gln His Glu Leu Leu Tyr Gln Lys Asn Trp Leu Glu
                165                 170                 175

Glu Glu Cys Ile Ala Trp Leu Lys Arg Phe Leu Glu Tyr Gly Lys Asp
            180                 185                 190

Thr Leu Gln Arg Thr Glu Ser Glu Thr Ile Pro Leu Val Met Lys Ala
        195                 200                 205

Val Ser Gly Ser Ile Val Leu Val Ile Val Leu Ala Gly Val Gly Val
    210                 215                 220

Leu Val Trp Arg Arg Arg Pro Arg Glu Gln Asn Gly Ala Ile Tyr Leu
225                 230                 235                 240

Pro Thr Pro Asp Arg
                245

<210>  5
<211>  341
<212>  PRT
<213>  Mus musculus

<400>  5

Met Met Leu Leu Leu Pro Leu Leu Ala Val Phe Leu Val Lys Arg Ser
1               5                   10                  15

His Thr Arg Thr His Ser Leu Arg Tyr Phe Arg Leu Ala Val Ser Asp
            20                  25                  30

Pro Gly Pro Val Val Pro Glu Phe Ile Ser Val Gly Tyr Val Asp Ser
        35                  40                  45

His Pro Ile Thr Thr Tyr Asp Ser Val Thr Arg Gln Lys Glu Pro Lys
    50                  55                  60

Ala Pro Trp Met Ala Glu Asn Leu Ala Pro Asp His Trp Glu Arg Tyr
65                  70                  75                  80

Thr Gln Leu Leu Arg Gly Trp Gln Gln Thr Phe Lys Ala Glu Leu Arg
                85                  90                  95

His Leu Gln Arg His Tyr Asn His Ser Gly Leu His Thr Tyr Gln Arg
            100                 105                 110

Met Ile Gly Cys Glu Leu Leu Glu Asp Gly Ser Thr Thr Gly Phe Leu
        115                 120                 125

Gln Tyr Ala Tyr Asp Gly Gln Asp Phe Ile Ile Phe Asn Lys Asp Thr
    130                 135                 140

Leu Ser Trp Leu Ala Met Asp Tyr Val Ala His Ile Thr Lys Gln Ala
145                 150                 155                 160

Trp Glu Ala Asn Leu His Glu Leu Gln Tyr Gln Lys Asn Trp Leu Glu
                165                 170                 175

Glu Glu Cys Ile Ala Trp Leu Lys Arg Phe Leu Glu Tyr Gly Arg Asp
            180                 185                 190

Thr Leu Glu Arg Thr Glu His Pro Val Val Arg Thr Thr Arg Lys Glu
        195                 200                 205

Thr Phe Pro Gly Ile Thr Thr Phe Phe Cys Arg Ala His Gly Phe Tyr
    210                 215                 220

Pro Pro Glu Ile Ser Met Thr Trp Met Lys Asn Gly Glu Glu Ile Ala
225                 230                 235                 240

Gln Glu Val Asp Tyr Gly Gly Val Leu Pro Ser Gly Asp Gly Thr Tyr
                245                 250                 255

Gln Thr Trp Leu Ser Val Asn Leu Asp Pro Gln Ser Asn Asp Val Tyr
            260                 265                 270

Ser Cys His Val Glu His Cys Gly Arg Gln Met Val Leu Glu Ala Pro
        275                 280                 285

Arg Glu Ser Gly Asp Ile Leu Arg Val Ser Thr Ile Ser Gly Thr Thr
    290                 295                 300

Ile Leu Ile Ile Ala Leu Ala Gly Val Gly Val Leu Ile Trp Arg Arg
305                 310                 315                 320

Ser Gln Glu Leu Lys Glu Val Met Tyr Gln Pro Thr Gln Val Asn Glu
                325                 330                 335

Gly Ser Ser Pro Ser
            340

<210>  6
<211>  271
<212>  PRT
<213>  Artificial Sequence

<220>
<223>  Soluble human MR1 isoform A

<400>  6

Arg Thr His Ser Leu Arg Tyr Phe Arg Leu Gly Val Ser Asp Pro Ile
1               5                   10                  15

His Gly Val Pro Glu Phe Ile Ser Val Gly Tyr Val Asp Ser His Pro
            20                  25                  30

Ile Thr Thr Tyr Asp Ser Val Thr Arg Gln Lys Glu Pro Arg Ala Pro
        35                  40                  45

Trp Met Ala Glu Asn Leu Ala Pro Asp His Trp Glu Arg Tyr Thr Gln
    50                  55                  60

Leu Leu Arg Gly Trp Gln Gln Met Phe Lys Val Glu Leu Lys Arg Leu
65                  70                  75                  80

Gln Arg His Tyr Asn His Ser Gly Ser His Thr Tyr Gln Arg Met Ile

85                  90                  95

Gly Cys Glu Leu Leu Glu Asp Gly Ser Thr Thr Gly Phe Leu Gln Tyr
            100                 105                 110

Ala Tyr Asp Gly Gln Asp Phe Leu Ile Phe Asn Lys Asp Thr Leu Ser
        115                 120                 125

Trp Leu Ala Val Asp Asn Val Ala His Thr Ile Lys Gln Ala Trp Glu
    130                 135                 140

Ala Asn Gln His Glu Leu Leu Tyr Gln Lys Asn Trp Leu Glu Glu Glu
145                 150                 155                 160

Cys Ile Ala Trp Leu Lys Arg Phe Leu Glu Tyr Gly Lys Asp Thr Leu
                165                 170                 175

Gln Arg Thr Glu Pro Pro Leu Val Arg Val Asn Arg Lys Glu Thr Phe
            180                 185                 190

Pro Gly Val Thr Ala Leu Phe Cys Lys Ala His Gly Phe Tyr Pro Pro
        195                 200                 205

Glu Ile Tyr Met Thr Trp Met Lys Asn Gly Glu Glu Ile Val Gln Glu
    210                 215                 220

Ile Asp Tyr Gly Asp Ile Leu Pro Ser Gly Asp Gly Thr Tyr Gln Ala
225                 230                 235                 240

Trp Ala Ser Ile Glu Leu Asp Pro Gln Ser Ser Asn Leu Tyr Ser Cys
                245                 250                 255

His Val Glu His Cys Gly Val His Met Val Leu Gln Val Pro Gln
            260                 265                 270

<210>  7
<211>  227
<212>  PRT
<213>  Artificial Sequence

<220>
<223>  Soluble human MR1 isoform B

<400>  7

Arg Thr His Ser Leu Arg Tyr Phe Arg Leu Gly Val Ser Asp Pro Ile
1               5                   10                  15

His Gly Val Pro Glu Phe Ile Ser Val Gly Tyr Val Asp Ser His Pro
            20                  25                  30

Ile Thr Thr Tyr Asp Ser Val Thr Arg Gln Lys Glu Pro Arg Ala Pro
        35                  40                  45

Trp Met Ala Glu Asn Leu Ala Pro Asp His Trp Glu Arg Tyr Thr Gln
    50                  55                  60

Leu Leu Arg Gly Trp Gln Gln Met Phe Lys Val Glu Leu Lys Arg Leu
65                  70                  75                  80

Gln Arg His Tyr Asn His Ser Gly Ser His Thr Tyr Gln Arg Met Ile
                85                  90                  95

Gly Cys Glu Leu Leu Glu Asp Gly Ser Thr Thr Gly Phe Leu Gln Tyr
            100                 105                 110

Ala Tyr Asp Gly Gln Asp Phe Leu Ile Phe Asn Lys Asp Thr Leu Ser
        115                 120                 125

Trp Leu Ala Val Asp Asn Val Ala His Thr Ile Lys Gln Ala Trp Glu
    130                 135                 140

Ala Asn Gln His Glu Leu Leu Tyr Gln Lys Asn Trp Leu Glu Glu Glu
145                 150                 155                 160

Cys Ile Ala Trp Leu Lys Arg Phe Leu Glu Tyr Gly Lys Asp Thr Leu
                165                 170                 175

Gln Arg Thr Glu Ser Glu Thr Ile Pro Leu Val Met Lys Ala Val Ser
            180                 185                 190

Gly Ser Ile Val Leu Val Ile Val Leu Ala Gly Val Gly Val Leu Val
        195                 200                 205

Trp Arg Arg Arg Pro Arg Glu Gln Asn Gly Ala Ile Tyr Leu Pro Thr
    210                 215                 220

Pro Asp Arg
225

<210>  8
<211>  198
<212>  PRT
<213>  Artificial Sequence

<220>
<223>  Soluble human MR1 isoform C

<400>  8

Arg Thr His Ser Leu Arg Tyr Phe Arg Leu Gly Val Ser Asp Pro Ile
1               5                   10                  15

His Gly Val Pro Glu Phe Ile Ser Val Gly Tyr Val Asp Ser His Pro
            20                  25                  30

Ile Thr Thr Tyr Asp Ser Val Thr Arg Gln Lys Glu Pro Arg Ala Pro
        35                  40                  45

Trp Met Ala Glu Asn Leu Ala Pro Asp His Trp Glu Arg Tyr Thr Gln
    50                  55                  60

Leu Leu Arg Gly Trp Gln Gln Met Phe Lys Val Glu Leu Lys Arg Leu
65                  70                  75                  80

Gln Arg His Tyr Asn His Ser Gly Ser His Thr Tyr Gln Arg Met Ile
                85                  90                  95

Gly Cys Glu Leu Leu Glu Asp Gly Ser Thr Thr Gly Phe Leu Gln Tyr
            100                 105                 110

Ala Tyr Asp Gly Gln Asp Phe Leu Ile Phe Asn Lys Asp Thr Leu Ser
        115                 120                 125

Trp Leu Ala Val Asp Asn Val Ala His Thr Ile Lys Gln Ala Trp Glu
    130                 135                 140

Ala Asn Gln His Glu Leu Leu Tyr Gln Lys Asn Trp Leu Glu Glu Glu
145                 150                 155                 160

Cys Ile Ala Trp Leu Lys Arg Phe Leu Glu Tyr Gly Lys Asp Thr Leu
                165                 170                 175

Gln Arg Thr Gly Lys Glu Lys Glu Lys Ala Ser Phe Pro His Cys Leu
            180                 185                 190

Asn Asn Cys Phe Tyr Thr
        195

<210>  9
<211>  227
<212>  PRT
<213>  Artificial Sequence

<220>
<223>  Soluble human MR1 isoform D

<400>  9

Arg Thr His Ser Leu Arg Tyr Phe Arg Leu Gly Val Ser Asp Pro Ile
1               5                   10                  15

His Gly Val Pro Glu Phe Ile Ser Val Gly Tyr Val Asp Ser His Pro
            20                  25                  30

Ile Thr Thr Tyr Asp Ser Val Thr Arg Gln Lys Glu Pro Arg Ala Pro
        35                  40                  45

Trp Met Ala Glu Asn Leu Ala Pro Asp His Trp Glu Arg Tyr Thr Gln
    50                  55                  60

Leu Leu Arg Gly Trp Gln Gln Met Phe Lys Val Glu Leu Lys Arg Leu
65                  70                  75                  80

Gln Arg His Tyr Asn His Ser Gly Ser His Thr Tyr Gln Arg Met Ile
                85                  90                  95

Gly Cys Glu Leu Leu Glu Asp Gly Ser Thr Thr Gly Phe Leu Gln Tyr
            100                 105                 110

Ala Tyr Asp Gly Gln Asp Phe Leu Ile Phe Asn Lys Asp Thr Leu Ser
        115                 120                 125

Trp Leu Ala Val Asp Asn Val Ala His Thr Ile Lys Gln Ala Trp Glu
    130                 135                 140

Ala Asn Gln His Glu Leu Leu Tyr Gln Lys Asn Trp Leu Glu Glu Glu
145                 150                 155                 160

Cys Ile Ala Trp Leu Lys Arg Phe Leu Glu Tyr Gly Lys Asp Thr Leu
                165                 170                 175

Gln Arg Thr Glu Ser Glu Thr Ile Pro Leu Val Met Lys Ala Val Ser
            180                 185                 190

Gly Ser Ile Val Leu Val Ile Val Leu Ala Gly Val Gly Val Leu Val
        195                 200                 205

Trp Arg Arg Arg Pro Arg Glu Gln Asn Gly Ala Ile Tyr Leu Pro Thr
    210                 215                 220

Pro Asp Arg
225

<210>  10
<211>  270
<212>  PRT

<213>  Artificial Sequence

<220>
<223>  Soluble mouse MR1 isoform A

<400>  10

Arg Thr His Ser Leu Arg Tyr Phe Arg Leu Ala Val Ser Asp Pro Gly
1               5                   10                  15

Pro Val Val Pro Glu Phe Ile Ser Val Gly Tyr Val Asp Ser His Pro
            20                  25                  30

Ile Thr Thr Tyr Asp Ser Val Thr Arg Gln Lys Glu Pro Lys Ala Pro
        35                  40                  45

Trp Met Ala Glu Asn Leu Ala Pro Asp His Trp Glu Arg Tyr Thr Gln
    50                  55                  60

Leu Leu Arg Gly Trp Gln Gln Thr Phe Lys Ala Glu Leu Arg His Leu
65                  70                  75                  80

Gln Arg His Tyr Asn His Ser Gly Leu His Thr Tyr Gln Arg Met Ile
                85                  90                  95

Gly Cys Glu Leu Leu Glu Asp Gly Ser Thr Thr Gly Phe Leu Gln Tyr
            100                 105                 110

Ala Tyr Asp Gly Gln Asp Phe Ile Ile Phe Asn Lys Asp Thr Leu Ser
        115                 120                 125

Trp Leu Ala Met Asp Tyr Val Ala His Ile Thr Lys Gln Ala Trp Glu
    130                 135                 140

Ala Asn Leu His Glu Leu Gln Tyr Gln Lys Asn Trp Leu Glu Glu Glu
145                 150                 155                 160

Cys Ile Ala Trp Leu Lys Arg Phe Leu Glu Tyr Gly Arg Asp Thr Leu
                165                 170                 175

Glu Arg Thr Glu His Pro Val Val Arg Thr Thr Arg Lys Glu Thr Phe
            180                 185                 190

Pro Gly Ile Thr Thr Phe Phe Cys Arg Ala His Gly Phe Tyr Pro Pro
        195                 200                 205

Glu Ile Ser Met Thr Trp Met Lys Asn Gly Glu Glu Ile Ala Gln Glu
    210                 215                 220

Val Asp Tyr Gly Gly Val Leu Pro Ser Gly Asp Gly Thr Tyr Gln Thr
225                 230                 235                 240

Trp Leu Ser Val Asn Leu Asp Pro Gln Ser Asn Asp Val Tyr Ser Cys
                245                 250                 255

His Val Glu His Cys Gly Arg Gln Met Val Leu Glu Ala Pro
            260                 265                 270

<210>  11
<211>  119
<212>  PRT
<213>  Homo sapiens

<400>  11

Met Ser Arg Ser Val Ala Leu Ala Val Leu Ala Leu Leu Ser Leu Ser
1               5                   10                  15

Gly Leu Glu Ala Ile Gln Arg Thr Pro Lys Ile Gln Val Tyr Ser Arg
            20                  25                  30

His Pro Ala Glu Asn Gly Lys Ser Asn Phe Leu Asn Cys Tyr Val Ser
        35                  40                  45

Gly Phe His Pro Ser Asp Ile Glu Val Asp Leu Leu Lys Asn Gly Glu
    50                  55                  60

Arg Ile Glu Lys Val Glu His Ser Asp Leu Ser Phe Ser Lys Asp Trp
65                  70                  75                  80

Ser Phe Tyr Leu Leu Tyr Tyr Thr Glu Phe Thr Pro Thr Glu Lys Asp
                85                  90                  95

Glu Tyr Ala Cys Arg Val Asn His Val Thr Leu Ser Gln Pro Lys Ile
            100                 105                 110

Val Lys Trp Asp Arg Asp Met
        115

<210>  12
<211>  119
<212>  PRT
<213>  Mus musculus

<400>  12

Met Ala Arg Ser Val Thr Leu Val Phe Leu Val Leu Val Ser Leu Thr
1               5                   10                  15

Gly Leu Tyr Ala Ile Gln Lys Thr Pro Gln Ile Gln Val Tyr Ser Arg

            20                  25                  30

His Pro Pro Glu Asn Gly Lys Pro Asn Ile Leu Asn Cys Tyr Val Thr
        35                  40                  45

Gln Phe His Pro Pro His Ile Glu Ile Gln Met Leu Lys Asn Gly Lys
    50                  55                  60

Lys Ile Pro Lys Val Glu Met Ser Asp Met Ser Phe Ser Lys Asp Trp
65                  70                  75                  80

Ser Phe Tyr Ile Leu Ala His Thr Glu Phe Thr Pro Thr Glu Thr Asp
                85                  90                  95

Thr Tyr Ala Cys Arg Val Lys His Ala Ser Met Ala Glu Pro Lys Thr
            100                 105                 110

Val Tyr Trp Asp Arg Asp Met
        115

<210> 13
<211> 99
<212> PRT
<213> Artificial Sequence

<220>
<223> Soluble human beta2 m

<400> 13

<210>  14
<211>  99
<212>  PRT
<213>  Artificial Sequence

<220>
<223>  Soluble mouse beta2 m

<400>  14